# EUROPEAN PATENT APPLICATION

(11) **EP 4 586 649 A1**
(43) Date of publication of application: **16.07.2025**
(21) Application number: 25179008.5
(22) Date of filing: 20.09.2022
(51) Int. Cl.: H04S 7/00, H04R 25/00, H04R 1/10

(54) **METHOD AND SYSTEM FOR MEASURING AND TRACKING EAR CHARACTERISTICS**

(30) Priority: 24.09.2021 US 202163248353 P; 24.08.2022 US 202217822064
(62) Divisional of application: 22196545.2
(71) Applicant: Apple Inc., Cupertino, CA 95014 (US)
(72) Inventor: KUSTER, Martin, Cupertino, 95014 (US); SAUX, Tom-Davy W., Cupertino, 95014 (US); MURGAI, Prateek, Cupertino, 95014 (US)
(74) Representative: COPA Copenhagen Patents

(57) **Abstract**

A method performed by a headset that includes a speaker and a set of in-ear microphones, the method includes driving, while the headset is being worn by a user, the speaker to project a first sound into a canal of a user's ear; receiving a first set of microphone signals from the set of in-ear microphones, the first set of microphone signals comprising the first sound and reflections of the first sound; generating at least one reflection parameter of the canal of the user's ear based on the first set of microphone signals; driving the speaker to project second sound into the canal of the user's ear; receiving a second set of microphone signals from the set of in-ear microphones, the second set of microphone signals comprising the second sound and reflections of the second sound; generating a set of output signals based on the second set of microphone signals and the at least one reflection parameter; and transmitting a notification related to a characteristic of a hearing element of the user's ear based on the set of output signals.

## Description

This application claims the benefit of U.S. Provisional Patent Application No. 63/248,353, filed on September 24, 2021, which application is incorporated herein by reference.

### FIELD

An aspect of the disclosure relates to a system that measures and tracks ear characteristics of one or more hearing elements of a user's ear. Other aspects are also described.

### BACKGROUND

Audiologists use special instruments for identifying, diagnosing, and treating issues with a person's ear. For instance, doctors may use tympanometry devices that test a person's middle ear's mobility due to changes in air pressure within the user's ear canal. These devices include a probe with a loudspeaker, a microphone, and air pump. With the probe inserted into the user's ear, the microphone measures sounds produced by the loudspeaker, while the air pump adjusts the air pressure within the user's ear. The input at the microphone is used to form a tympanogram, from which doctors may determine various issues with the user's ear.

### SUMMARY

An aspect of the disclosure is a method performed by a headset, such as a pair of in-ear headphones (e.g., earbuds), over-the-ear headphones, or on-the-ear headphones, which is arranged to be worn by (e.g., on a head of) a user and includes a speaker and an in-ear (or error) microphone. The headset performs a calibration on the headset to obtain a baseline measurement. For instance, the calibration may be performed while the headset is in a controlled environment (e.g., before it is worn by the user), such as a holding case that is arranged to house the headset while not worn by the user. The headset uses, while the headset is being worn by the user, an audio signal (e.g., which may include user-desired audio content, such as music, and/or may include one or more test sounds) to drive the speaker that is arranged to project sound into a canal of the user's ear. The headset captures as a microphone signal, from the in-ear microphone of the headset, sound from within the canal of the user's ear. The headset determines a parameter associated with the user's ear based at least on the captured microphone signal and the baseline measurement. The headset transmits a notification related to one or more characteristics of hearing elements of the user's ear based on the parameter.

In one aspect, the headset uses the microphone signal to determine a secondary path (or S-path) transfer function that represents a response between the speaker and the in-ear microphone (while both components are inserted inside the user's ear canal), where the parameter may be based on the S-path transfer function. In some aspects, the transfer function may be determined as part of an active-noise cancellation (ANC) process that is performed by the headset to generate an anti-noise signal for driving the speaker. In another aspect, however, the headset may not be configured to perform the ANC process (e.g., may not have ANC functionality), in which case, the S-path transfer function may not be determined as part of (or may be determined separate from) an ANC process.

In one aspect, the parameter is determined by measuring, using the S-path transfer function, an acoustic input impedance of the user's ear canal with respect to the baseline measurement and using the acoustic impedance to determine the parameter that is part of an equivalent acousto-mechanical model of hearing elements of the user's ear, such as at least the user's middle ear and outer ear. In another aspect, the headset compares the parameter to a corresponding parameter of a predefined equivalent acousto-mechanical model, and identifies the one or more characteristics based on a difference between the parameter and the corresponding parameter of the predefined model. In some aspects, the acoustic input impedance is measured over a period of time, and the headset determines the one or more characteristics based on a change between the parameter and the corresponding parameter over the period of time. In another aspect, the parameter may be a reflection mask of the canal of the user's ear that is determined based on the acoustic impedance. In which case, the headset may filter one or more microphone signals using the reflection mask to produce one or more filtered signals that are used to determine a severity level of a characteristic.

In some aspects, performing the calibration comprises determining one or more impedance calibration parameters for an impedance model, where measuring the acoustic input impedance includes estimating the impedance by applying the determined one or more impedance calibration parameters and the S-transfer function to the impedance model. In another aspect, the one or more impedance calibration parameters are parameters that are predefined offline in a controlled environment.

In another aspect, determining the parameter associated with the user's ear includes measuring, using the S-path transfer function, an acoustic input impedance of the user's ear canal with respect to the baseline measurement as the parameter, and determining the one or more characteristics of hearing elements of the user's ear based on the parameter includes using the acoustic impedance to determine that there is a blockage within the canal of the user's ear and determine a severity level of the blockage.

In one aspect, the audio signal includes user-desired audio content. In another aspect, the audio signal includes a (e.g., predefined) test sound. In some aspects, the calibration is performed on the headset before using the audio signal to drive the speaker and before the capturing of the sound form within the canal of the user. In one aspect, the calibration is performed on the headset prior to the headset being worn by the user. In some aspects, the calibration is performed on the headset while the headset is in a controlled environment. In one aspect, the calibration is performed while the headset is in a holding case that is arranged to house the headset while it is not worn by the user. In another aspect, the one or more characteristics are determined using a machine learning model that has an input based on the parameter.

According to another aspect of the disclosure, a method performed by the headset may use one or more microphone signals to determine a characteristic of a hearing element of the user's ear. For instance, the headset may drive, while the headset is being worn by the user, the speaker to project first sound into a canal of the user's ear, and receive a first set of microphone signals from a set of in-ear microphones, the first set of microphone signals including the first sound (e.g., as direct sound from the speaker) and reflections of the first sound (e.g., reflections off of the user's ear canal, ear drum, etc.) captured by the microphones. The headset generates at least one reflection parameter of the canal of the user's ear based on the first set of microphone signals. The headset drives the speaker to project second sound into the canal of the user's ear, and receives a second set of microphone signals that includes the second sound and reflections of the second sound. The headset generates a set of output signals based on the second set of microphone signals and the at least one reflection parameter, where the output signals include only the reflections of the second sound. The headset transmits a notification related to a characteristic of a hearing element of the user's ear based on the set of output signals.

In one aspect, generating the at least one reflection parameter comprises determining whether spectral content across a frequency band of a plurality of frequency bands of the first set of microphone signals primarily includes the first sound as direct sound from the speaker or the reflections of the first sound based on a comparison of the set of first microphone signals, wherein the at least one reflection parameter is generated in response to the spectral content including the reflections of the first sound. In another aspect, determining whether the spectral content primarily includes the first sound as direct sound from the speaker or the reflections of the first sound comprises determining, over a period of time, whether the spectral content across the frequency band of the first set of microphone signals includes a threshold number of reflections of the first sound.

In some aspects, each of the at least one reflection parameter is associated with a respective frequency band of the plurality of frequency bands, wherein generating the set of output signals comprises filtering, using the at least one reflection parameter, the second sound from the second set of microphone signals by removing spectral content across one or more frequency bands that are not associated with the at least one reflection parameter. In another aspect, the headset uses the first set of microphone signals to produce a directional beam pattern that is directed towards the canal of the user's ear and away from the headset, wherein the spectral content is of the directional beam pattern.

In one aspect, the headset determines whether the first sound exceeds a confidence threshold based on audio content of the first sound; and in response to the first sound exceeds the confidence threshold, the notification related to the characteristic of the hearing element is transmitted. In some aspects, determining whether the first sound exceeds a confidence threshold comprises determining whether the audio content includes at least one of a decay and a pause.

In some aspects, the characteristic is a blockage within the canal of the user's ear and the ear geometry is a geometry of the canal, the headset estimates, using the set of output signals, a geometry of the blockage within the canal, the severity level is based on one or more differences between the geometry of the canal and the geometry of the blockage within the canal.

The above summary does not include an exhaustive list of all aspects of the disclosure. It is contemplated that the disclosure includes all systems and methods that can be practiced from all suitable combinations of the various aspects summarized above, as well as those disclosed in the Detailed Description below and particularly pointed out in the claims. Such combinations may have particular advantages not specifically recited in the above summary.

### BRIEF DESCRIPTION OF THE DRAWINGS

The aspects are illustrated by way of example and not by way of limitation in the figures of the accompanying drawings in which like references indicate similar elements. It should be noted that references to "an" or "one" aspect of this disclosure are not necessarily to the same aspect, and they mean at least one. Also, in the interest of conciseness and reducing the total number of figures, a given figure may be used to illustrate the features of more than one aspect, and not all elements in the figure may be required for a given aspect.
**Fig. 1** shows a system that includes an output device according to one aspect.
**Fig. 2** shows a block diagram of the output device inserted into a user's ear and is configured to determine one or more characteristics of the user's ear according to one aspect.
**Fig. 3** is a flowchart of one aspect of a process for determining characteristics of a user's ear.
**Fig. 4** shows another block diagram of the output device that is configured to determine a characteristic of the user's ear based on one or more reflection parameters of the user's ear canal from one or more microphone signals according to one aspect.
**Fig. 5** is a flowchart of one aspect of a process for determining one or more characteristics of the user's ear based on one or more estimated reflection parameters of the user's ear canal.
**Fig. 6** shows another block diagram of the output device that is configured to determine a characteristic of a user's ear based on an acoustic input impedance measurement according to one aspect.

### DETAILED DESCRIPTION

Several aspects of the disclosure with reference to the appended drawings are now explained. Whenever the shapes, relative positions and other aspects of the parts described in a given aspect are not explicitly defined, the scope of the disclosure here is not limited only to the parts shown, which are meant merely for the purpose of illustration. Also, while numerous details are set forth, it is understood that some aspects may be practiced without these details. In other instances, well-known circuits, structures, and techniques have not been shown in detail so as not to obscure the understanding of this description. Furthermore, unless the meaning is clearly to the contrary, all ranges set forth herein are deemed to be inclusive of each range's endpoints.

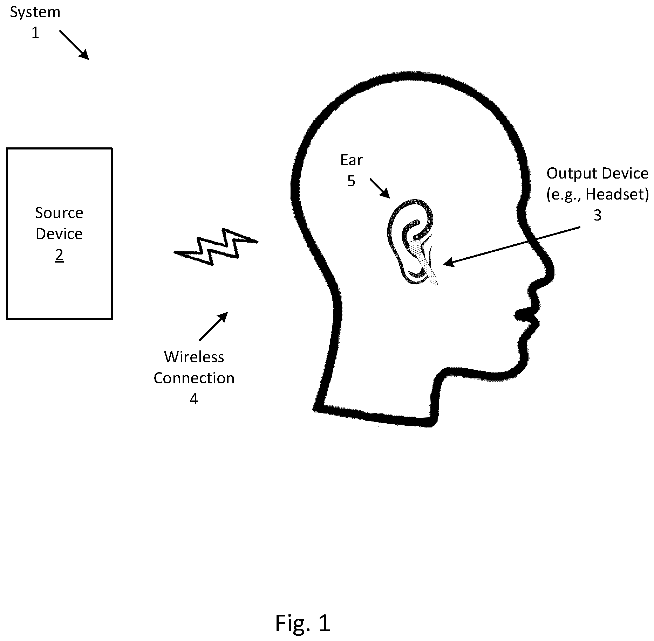

**Fig. 1** shows a system 1 that includes a source device 2 and an output device 3 according to one aspect. In one aspect, the system may include other devices, such as a remote electronic server (not shown) that may be communicatively coupled to the source device, the audio device, or both devices, and may be configured to perform one or more operations as described herein. In one aspect, the source device 2 and/or output device 3 may perform at least some of the operations described herein. In another aspect, the system may include fewer devices, such as only having the output device 3. In which case, the output device may perform at least some of the operations described herein.

In one aspect, the source device 2 may be any electronic device (e.g., that includes one or more processors, memory, etc.) that can perform audio signal processing operations and/or networking operations. Examples of sources devices may include a desktop computer, a smart speaker, a home theater system, or an infotainment system that is integrated within a vehicle, etc. In some aspects, the source device may be a wireless electronic device, such as a tablet computer, a smart phone, etc. In another aspect, the source device may be a wearable device, such as a smart watch, and/or may be a head-mounted device (HMD), such as smart glasses, etc.

As illustrated, the output device 3 is a headset (e.g., a pair of in-ear headphones or earbuds) that are positioned next to (against or inside) the user's ear 5. In particular, the device is illustrated as being a right earbud that is against (or positioned on) the user's right ear and/or is at least partially inserted inside the user's ear canal. In one aspect, the headset may include another earbud (e.g., a left earbud) that is positioned against the user's left ear. In which case, the output device may perform one or more audio signal processing operations within at least one of (or both of) the earbuds. In the case in which one earbud performs at least some of the operations described herein, the earbud may produce one or more driver signals, and transmit at least one driver signal to the other earbud, so that both earbuds may drive one or more respective speakers. In another aspect, both earbuds may perform one or more (e.g., similar) operations to produce individual driver signals.

In one aspect, once the headset (or in-ear headphone) is placed against the user's ear, the headset may acoustically seal off the user's ear (canal) from the ambient environment, thereby preventing (or reducing) sound leakage into (and out of) the user's ear (and/or ear canal). Specifically, a housing of the headset may be placed onto, around, and/or into the user's ear in order to prevent (or reduce) sound leakage into (and out of) the user's ear (ear canal). For example, when the headset is in-ear headphones, the headphones may be a sealing-type headphones in which a housing of the headphones includes a portion, such as an ear tip for each of the user's ears, where each of the ear tips is arranged to create a seal within (or seal off) the ear canal of the user when inserted into one of the user's ears.

In some aspects, the output device 3 may be any type of headphones, such as on-ear or over-the-ear headphones. In the case of over-the-ear headphones, the output device may be a part of a headphone housing that is arranged to cover at least a portion of the user's ears to (at least partially) seal off the ear (and ear canal) from the ambient environment. In another aspect, may be any type of electronic device that is arranged to be positioned onto (or in) the user's ears for sound output, such as a hearing aid. In some aspects, the output device may be any (or a part of any) HMD, such as smart glasses. For instance, the audio output device may be a part of a component (e.g., a frame) of the smart glasses that may be placed into or on the user's ears. In another aspect, the output device may be a HMD that (at least partially) does not cover the user's ears (or ear canals), thereby leaving the user's ears exposed to the ambient environment.

In one aspect, the output device 3 may be any electronic device that is configured to output sound, performing networking operations, and/or perform audio signal processing operations, as described herein. For example, the output device may be configured to receive one or more audio signals (e.g., from the source device), and may be configured to use the one or more audio signals to drive one or more speakers to output sound directed towards the user's ears and/or ear canals. More about driving speakers is described herein. In one aspect, the audio output device may be a device that is not designed to be positioned next to (or against) the user's ears. For example, the output device may be a (e.g., stand-alone) loudspeaker, a smart speaker, a part of a home theater system, a part of an infotainment system that is integrated within a vehicle, etc. In some aspects, the output device may be a part of another electronic device, such as a laptop, a desktop, or a multimedia device. In another aspect, the output device and the source device may be the same devices.

In one aspect, the output device 3 may be configured to be wirelessly communicatively coupled to (or with) one or more other electronic devices (e.g., the source device 2), such that both devices are configured to communicate with one another. For example, the output device 3 is communicatively coupled with the source device via a wireless connection 4. For example, the wireless output device, such as a wireless headset or a pair of wireless earphones, can connect via a Wireless Personal Area Network (WPAN) connection to the source device, in order to receive an audio stream from the audio source device. In one aspect, the WPAN connection may be via an Advanced Audio Distribution Profile (A2DP) connection or another audio profile connection of a BLUETOOTH communication protocol. To stream high-quality audio data, the source device packetizes the audio data (e.g., partitions the audio data into units for transmission) according to the audio profile and stores the packets of audio data in transmit buffers. Packets are then transmitted over BLUETOOTH (e.g., using an over-the-air (or wireless) radio frequency (RF) signal) to the wireless output device. The received packets are stored in long buffers in the output device in order to provide continuous audio playback in situations when future packets are dropped (e.g., during transmission interference). Audio data in the buffers are de-packetized and processed for audio output through at least one speaker. This process is repeated while audio output is desired at the audio output device.

In another aspect, the wireless connection 4 between the output device and the source device may be via one or more other wireless computer networks. For example, the output device may communicatively couple with the source device via any network, such as a wirelessly local area network (WLAN), a wide area network (WAN), a cellular network, etc., in order to exchange digital (e.g., audio) data. With respect to the cellular network, the output device may be configured to establish a wireless (e.g., cellular) call, in which the cellular network may include one or more cell towers, which may be part of a communication network (e.g., a 4G Long Term Evolution (LTE) network) that supports data transmission (and/or voice calls) for electronic devices, such as mobile devices (e.g., smartphones).

In another aspect, the output device 3 may communicatively couple with the source device 2 via other methods. For example, both devices may couple via a wired connection. In this case, one end of the wired connection may be (e.g., fixedly) connected to the output device, while another end may have a connector, such as a media jack or a universal serial bus (USB) connector, which plugs into a socket of the source device. Once connected, the output device may be configured to drive one or more speakers of the output device with one or more audio signals, via the wired connection. For instance, the source device may transmit the audio signals as digital audio (e.g., PCM digital audio). In another aspect, the audio may be transmitted in analog format.

In some aspects, the output device 3 and the source device 2 may be distinct (separate) electronic devices, as shown herein. In another aspect, the output device may be a part of (or integrated with) the source device. For example, as described herein, at least some of the components of the output device (such as a controller) may be part of the source device, and/or at least some of the components of the source may be part of the output device. In this case, each of the devices may be communicatively coupled via traces that are a part of one or more printed circuit boards (PCBs) within the device.

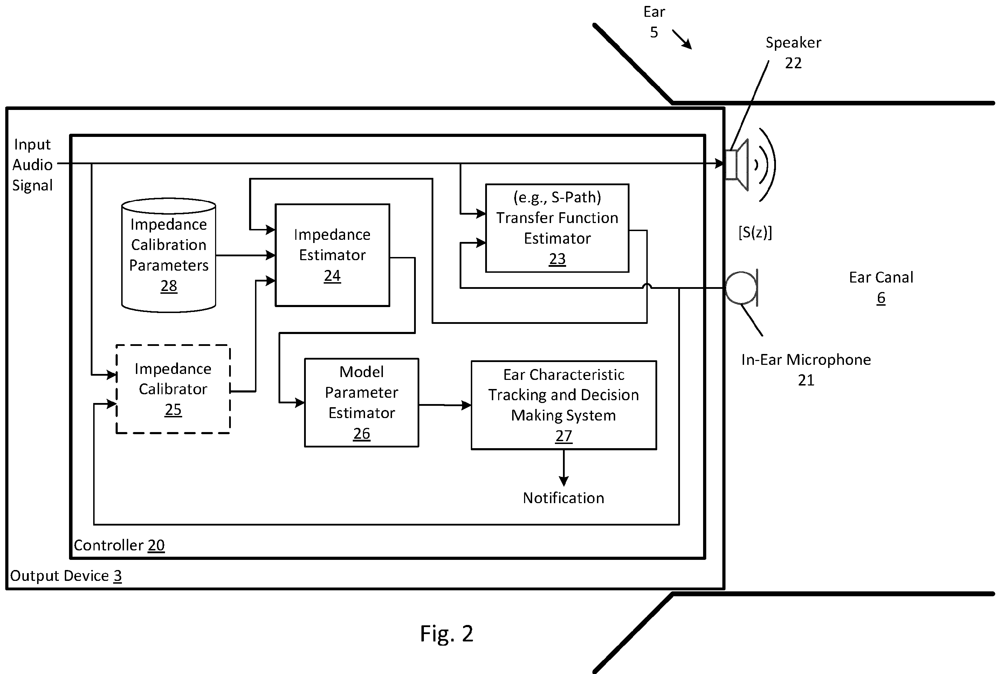

**Fig. 2** shows a block diagram of the output device 3 inserted into a user's ear 5 and is configured to determine one or more characteristics of one or more hearing elements of the user's ear according to one aspect. Specifically, as shown at least a portion of the output device is inserted into the ear canal 6 of the user's ear 5. As described herein, when inserted into the ear, the output device may create a seal within the ear canal, to prevent air movement (e.g., leakage) between the ear canal 6 and the ambient environment (e.g., outside the ear canal). For instance, the output device may be an earbud with an ear tip (e.g., coupled to a housing of the earbud), which is arranged to create a seal (e.g., by coming into contact with an inner wall of the ear canal) within the canal, as described herein. In another aspect, the output device 3 may be resting on the user's ear 5, such as on-ear (or over-the-ear) headphones, as described herein.

As described herein, the output device is configured to determine and transmit (e.g., output) one or more characteristics of hearing elements of the user's ear. Specifically, the output device may perform operations to determine (detect or identify) characteristics (e.g., issues, concerns, problems, etc.) with hearing elements that may adversely affect a user's ability to hear sounds. In one aspect, a detected characteristic may be specific types of issues (e.g., clinical disorders) with a hearing element of a user's ear. In another aspect, however, characteristics as described herein may be a general indication of a problem or issue with one or more hearing elements, rather than being a specific clinically defined issue. As used herein, the hearing elements may include parts of the user's middle ear (e.g., tympanic cavity), such as the malleus, incus, and/or stapes, and/or may include parts of the user's inner ear, such as the cochlea. As another example, a hearing element may include the outer ear (e.g., the auditory canal or ear canal), or the tympanic membrane (or ear drum). In another aspect, a hearing element may represent a (or any) physical part of the user's ear.

In one aspect, a characteristic with a hearing element may be the result of a natural occurrence, or may be the result of an external force (e.g., that results in damage to or a reduction in (audible) performance of one or more hearing elements). For instance, the external force may be an object interacting (or coming into contact) with a hearing element, such as a blockage within the user's ear canal. The blockage within the user's ear (e.g., ear canal) may result in at least a partial hearing loss due to the passage of sound through the user's ear canal being at least partially blocked. More about blockages is described herein. In another aspect, a characteristic may be a disorder (or a particular issue) with a particular hearing element.

Conventionally, a person may wait to schedule an appointment with a medical professional (e.g., a doctor) for a consultation, when symptoms associated with the user's hearing (or ears) occur. For example, a person may contact an audiologist, once symptoms occur with the person's hearing and/or ear (e.g., a loss of hearing, ear pain, dizziness, headaches, etc.). Once at the appointment, the audiologist may use specialized tools to perform hearing tests. For instance, to identify a condition with a person's ear, the audiologist may perform Tympanometry tests in which a Tympanometry probe is inserted into a person's ear canal, and the probe measures changes in air pressure. Although the audiologist may be able to diagnose the problem, the person may inadvertently exacerbate the condition by waiting too long to receive the consultation and may thereby unnecessarily experience the symptoms over an extended period of time.

To overcome these deficiencies, the present disclosure describes an output device that is configured to detect and track characteristics with one or more hearing elements of the user's ear. Specifically, the output device may be a headset, such as an in-ear headphone (e.g., earbuds) that includes a speaker and an in-ear microphone (or error microphone). The output device may use, while the output device is being worn by the user (e.g., the device being inserted into or on the user's ear canal), an audio signal to drive the speaker to project sound into the ear canal, and may capture a microphone signal that includes sound from within the ear canal. The output device may determine a parameter associated with the user's ear based at least on the captured microphone signal. Based on the parameter, the output device may determine one or more characteristics of hearing elements of the user's ear. In addition, the output device may transmit (present or output) a notification (e.g., which may be audible and/or visual) to the user indicating that a characteristic has been detected. Thus, the present disclosure provides an output device, such as an in-ear headphone, as opposed to a specialized medical tool, which is able to notify a user of one or more detected characteristics with the user's ear. The user may be able to schedule a consultation with the medical professional, in response to the notification, in order to receive a medical diagnosis. As a result, the user may be able to receive a consultation sooner than if not otherwise notified of the characteristic, which may reduce the amount of time the person has to experience symptoms.

Returning to **Fig. 2****,** the output device 3 includes a controller 20, a speaker 22, and an in-ear (or error) microphone 21. In one aspect, the output device may include more (or less) elements (or components). For example, the output device may include one or more speakers, microphones, etc. In another aspect, the output device may include other components (not shown), such as a reference microphone, one or more display screens, and one or more sensors (e.g., an accelerometer, an inertial measurement unit (IMU), a camera, etc.).

The controller 20 may be a special-purpose processor such as an application-specific integrated circuit (ASIC), a general purpose microprocessor, a field-programmable gate array (FPGA), a digital signal controller, or a set of hardware logic structures (e.g., filters, arithmetic logic units, and dedicated state machines). The controller is configured to perform audio signal processing operations and/or networking operations. For instance, the controller 20 may be configured to perform ear characteristic tracking and decision making operations, as described herein. In another aspect, the controller may be configured to perform other audio signal processing operations. More about the operations performed by the controller 20 is described herein.

The in-ear (or error) microphone 21 may be any type of microphone (e.g., a differential pressure gradient micro-electro-mechanical system (MEMS) microphone) that is configured to convert acoustical energy caused by sound wave propagating in an acoustic environment into an input microphone signal. As described herein, the microphone is an in-ear microphone, such that when the output device is worn by (e.g., on a head of) a user, the microphone may be positioned (or arranged) to sense sound (e.g., differences in air pressure) within (or near) the user's ear canal. As shown in this figure, the microphone is at an end of the output device that has been inserted in the user's ear canal 6. Thus, the in-ear microphone may be positioned inside or at the entrance to the ear canal 6 in order to capture sound from inside or near the user's ear.

The speaker 22 may be an electrodynamic driver that may be specifically designed for sound output at certain frequency bands, such as a woofer, tweeter, or midrange driver, for example. In one aspect, the speaker 22 may be a "full-range" (or "full-band") electrodynamic driver that reproduces as much of an audible frequency range as possible. In one aspect, when the output device includes one or more speakers, each of the speakers may be the same type (e.g., each being a full-range driver), or may be of different types. For example, when the output device includes two speakers, one may be a midrange driver, while another is a woofer.

As shown in this figure, the speaker 22 is an "internal" speaker that is configured to project sound into (or towards) a user's ear canal, while the output device is being used (or worn) by the user. Thus, similar to the microphone 21, the speaker may be positioned inside (or at the entrance to) the ear canal 6 of the user's ear 5 in order to project the sound inward into the ear canal. In another aspect, the speaker may be an "extra-aural" speaker that is arranged to output sound into the ambient environment. Similarly, the output device may include one or more "reference" microphones that are each arranged to sense sound from within the ambient environment. In another aspect, the output device may include one or more of each (or some) of these speakers/microphones. For instance, the output device may include one or more internal speakers (e.g., speaker 22) and include one or more extra-aural speakers.

In one aspect, each (or at least some) of the components of the output device 3 may be integrated into (e.g., a housing of) the output device, such that the components are a part of the device. In another aspect, one or more of the components may be a part of separate electronic devices that are communicatively coupled with the output device. For example, one or more reference microphones may be a part of the source device 2, which is configured to capture ambient sound with the reference microphones and may be configured to transmit microphone signals captured by the microphones to the output device (e.g., for audio signal processing operations).

As described herein, the controller 20 is configured to perform ear characteristic tracking and decision making operations. Specifically, the controller includes one or more operational blocks to perform these one or more of these operations. As shown, the controller includes a (e.g., secondary path or S-path) transfer function estimator 23, an impedance estimator 24, an impedance calibrator 25 (which is optional, as illustrated by having a dashed boundary), a model parameter estimator 26, an ear characteristic tracking and decision making system 27, and impedance calibration parameters 28. In one aspect, the controller may have more or less operational blocks. For example, the controller may not include the optional impedance calibrator.

In one aspect, at least some of the operations performed by one or more of the operational blocks may occur while the (e.g., controller 20 of the) output device is performing one or more audio signal processing operations. For instance, the controller is receiving an input audio signal (e.g., as an analog or digital audio signal), and may be using the audio signal to drive the speaker 22 that is arranged to project sound of the audio signal into the ear canal 6 of the user. Thus, the controller may be driving the speaker while the output device 3 is being worn by (e.g., at least partially inserted into ear canal of) the user.

In one aspect, the input audio signal may include media content or user-desired audio content, such as a musical composition, a podcast, a movie soundtrack, etc. In another aspect, the input audio signal may include a predefined test sound (e.g., a sine sweep), which may have been defined in a controlled environment (e.g., a laboratory), and may be used to perform to determine one or more characteristics of the user's ear. In one aspect, the controller may be receiving (or obtaining) the input audio signal from local memory. In another aspect, the controller may be receiving the audio signal from a remote device, such as the source device 2. In which case, the output device may stream (e.g., via the wireless connection 4) the input audio signal for playback by the output device.

In one aspect, the audio signal may include sounds from one or more software applications that are being executed by the (e.g., controller 20 of the) audio output device. For instance, the audio signal may be a downlink signal that is a part of a telephone call being conducted by a telephony application (which is being executed by the output device and/or the source device). In which case, the telephony application may be configured to exchange audio data (e.g., via the source device) over a communications network with another device with which the output device is conducting the call. In another aspect, the audio signal may include other sounds of other software applications, such as having an audible alert that is associated with a virtual personal assistant application. In one aspect, these applications may be executed by the source device, while the sounds of the software application are being transmitted (via the wireless connection 4) to the output device for playback.

In one aspect, the input audio signal may be a single audio signal (e.g., a mono audio signal). In another aspect, the controller may obtain two or more input audio signals, such as obtaining two audio channels (e.g., a stereo signal) for output through two or more speakers. In which case, the output device may include two or more speakers, such as having two or more internal speakers (e.g., speakers such as speaker 22 that is arranged to project sound into the user's ear canal, while the output device is worn by (or on) a user. In another aspect, at least some of the audio signals may be for driving one or more extra-aural speakers. More about other audio signal processing operations that may be performed by the controller is described herein.

The transfer function estimator 23 is configured to receive the input audio signal that is being used (or is going to be used) to drive the speaker 22 and a microphone signal that includes sound from within the ear canal 6 that is captured by the in-ear microphone 21, and is configured to estimate (measure or determine) a transfer function that represents a travel path (or response) between the speaker 22 and the in-ear microphone, using the input audio signal and the microphone signal. Thus, the transfer function is estimated from the air pressure measured by the in-ear microphone. In another aspect, the transfer function is estimated by measuring the ear canal impedance from the pressure at the in-ear microphone and one or more characteristics of the speaker (e.g., a volume velocity of the speaker). In one aspect, a response, and an impulse response as used herein are equivalents of one another. In some aspects, the impulse response is the response of the in-ear microphone signal in the time domain. In another aspect, the transfer function is the frequency response of the impulse response, and is in the frequency domain.

In one aspect, the transfer function may be determined as part of an active-noise cancellation (ANC) process (or function) that is performed by the controller 20 of the output device to generate an anti-noise signal for driving the speaker 22. For instance, the controller may be configured to perform adaptive feedback ANC to adapt an ANC filter (e.g., *W(z)*) according to an estimate of a secondary path (or S-path) transfer function (*S(z)*) that represents the travel (or acoustic) path from the speaker 22 and the microphone 21 (as shown in this figure), which the ANC process uses to adjust an adaptive digital filter used to produce the anti-noise signal. In one aspect, the S-path transfer function defines how the microphone output signal responds to an impulse input to the speaker. In some aspects, the S-path transfer function may be estimated by a fixed transfer function, or it may be adaptively computed online by an adaptive filter algorithm while the device (e.g., headphone) is being worn by a user. In one aspect, the S-path transfer function may be estimated, while the ANC function is activated (e.g., based on user input received by the output device).

As described thus far, the controller may be configured to perform feedback ANC. In another aspect, in addition to (or in lieu of) performing feedback operations, the controller may perform a feedforward ANC function in which a reference microphone signal captured by a reference microphone (not shown) that is arranged to capture ambient sound (and the error microphone signal) are used to adapt an ANC filter according to the S-path transfer function in order to produce another anti-noise signal.

In another aspect, the transfer function estimator 23 may estimate the S-path transfer function separate from (or not determined as part of) the ANC process. For example, the output device may not be configured to perform the ANC process (e.g., the controller not being configured to perform the process), but may still be configured to estimate the S-path. In another aspect, the S-path transfer function may be estimated as part of the ANC process, while the ANC process is not generating the anti-noise signal. For example, the controller may (e.g., only) be executing the ANC process to estimate the transfer function.

The impedance estimator 24 is configured to receive the (e.g., S-path) transfer function from the transfer function estimator 23 and is configured to measure (or estimate) an acoustic input impedance of the ear canal 6 using the S-path transfer function. For example, the impedance estimator may use the transfer function to model the acoustic input (e.g., ear drum) impedance (e.g., and the ear canal, separately). Specifically, the impedance estimator may be configured to receive impedance calibration parameters (or coefficients) 28 (e.g., which may be stored in memory of the controller), and apply the parameters and the S-path transfer function upon an impedance model (e.g., stored within memory) to produce (or estimate) the acoustic input impedance. Specifically, the coefficients may be used to translate the S-path transfer function to the acoustic input impedance (e.g., according to the impedance model). In some aspects, the impedance calibration parameters may define a calibration curve (e.g., which may include one or more curves of the coefficients with respect to frequency). In which case once the impedance estimator may use the calibration curve (with respect to the S-path transfer function) to estimate the acoustic input impedance of the sound into the user's ear canal. In one aspect, the impedance calibration parameters (which may be one or more frequency dependent parameters) may be determined (or measured) via an impedance calibration operation (or measurement) that is performed offline in a controlled environment, such as during product development, in a power-up cycle.

In one aspect, the impedance calibrator 25 may be configured to perform a calibration of the output device to obtain a baseline (or calibration) measurement. For instance, the impedance calibrator may be configured to perform the offline impedance calibration operation in order to determine the impedance calibration parameters. In another aspect, the stored parameters may be received from another electronic device that performs the calibration. In another aspect, the impedance calibrator is configured to perform an (e.g., on-line) impedance calibration to account for drift (or variance) between one or more acoustic components (e.g., between speakers and/or microphones) over time. In one aspect, variances may occur due to natural degrading of the components over time and/or may be based on changes to environmental conditions, such as objects or pollutants collecting on or near components over time (e.g., dust collecting in speaker ports). For example, the impedance calibrator may be configured to receive the input audio signal and the microphone signal from the in-ear microphone, and may be configured to perform the baseline measurement, which may be an impulse response measurement (e.g., transfer function) of output sound of the speaker 22 at a location of the in-ear microphone. In one aspect, the baseline measurement may be performed before (or after) the output device is worn by the user. Specifically, this measurement may be performed while the output device (or at least a portion of the output device that includes the speaker and the microphone) is in a controlled environment, such as in a container that is designed (or arranged) to house the output device while it is not being used by a user. For example, when the output device is a headset (e.g., pair of earbuds), the container may be a holding case that is designed to house the headset while it is not being worn by the user. Thus, the output device may perform an impedance calibration while inside the case in order to account for the drift. In one aspect, this impedance calibration may involve estimating the S-path transfer function, and determining whether one or more of the impedance calibration parameters 28 has changed (e.g., from parameters that were determined offline). For example, the estimated S-path transfer function may be associated with a predefined acoustic input impedance that is associated with the volume of the case. Changes between the recently determined acoustic input impedance (taken while in the case) and the predefined impedance may be the result of the drift, and as a result, the impedance calibrator may adjust one or more of the parameters 28 based on the drift. In another aspect, in addition to (or in lieu of) adjusting parameters, the impedance calibrator may be configured to use the calibration to adjust a (e.g., future) estimated transfer function to account for the changes to the acoustic components.

In one aspect, the impedance calibrator may be configured to periodically obtain (or perform) one or more baseline measurements (e.g., impedance recalibrations). For instance, the calibrator may periodically (e.g., once a day, a week, a month, etc.) perform the impedance calibration in order to maintain an optimal impedance estimation. In another aspect, the calibrator may perform the measurement upon determining that the output device is in a controlled environment (e.g., based on sensor data captured by one or more sensors of the output device, which indicate that the output device has been placed in a holding case, as described herein). In another aspect, the calibration measurement may be performed based on user input (e.g., based on a user command received by the output device).

In another aspect, the impedance calibrator may be configured to perform the baseline measurement, while the output device is being used (e.g., worn) by the user. Specifically, the impedance calibrator may perform this measurement periodically in order to track changes in impedance over time (e.g., from when the output device was newly purchased by the user to a present time).

In one aspect, the impedance estimator may be configured to receive the baseline measurement (e.g., the impedance calibration of the impedance calibrator), and may be configured to measure (or estimate) the acoustic input impedance of the user's ear canal, using the transfer function and with respect to the baseline measurement. For example, the impedance estimator may be configured to compensate for changes in impedance (e.g., based on variance, as described herein) by adjusting (or compensating) the impedance calibration parameters 28 and/or the estimated S-path transfer function based on the impedance calibration. Thus, the impedance estimator may use the (e.g., one or more) baseline measurement(s) to account for variance when estimating the acoustic input impedance of the user's ear canal.

In one aspect, one or more hearing elements of a physical ear may be modeled (or estimated) as an equivalent acousto-mechanical model. For example, the equivalent model may be an RLC circuit (or network), where hearing elements of the physical ear are modeled as one or more parameters (or components) of the RLC network. Specifically, hearing elements such as the outer ear, eardrum, middle ear, and/or inner ear may be modeled as one or more electrical and/or mechanical components of the RLC network (e.g., a resistor, a capacitor, an inductor, a damper, etc.). In one aspect, one or more of these four parts of the human ear may be composed of one or more components that make up the model. For example, the ear canal of the outer ear may be modeled as a damper, while the cochlea of the inner ear may be modeled as an inductor. In one aspect, the modeled hearing elements may also include characteristics of one or more of the elements. For example, the RLC circuit may model a characteristic of the stapes of the middle ear (e.g., where a stiffness of the stapes may be represented by a capacitor). In another aspect, the equivalent model may be composed of any type of electrical component and/or mechanical component.

The model parameter estimator 26 is configured to receive the estimated acoustic input impedance from the impedance estimator 24 and is configured to use the acoustic input impedance to determine (e.g., at least a portion of) an equivalent acousto-mechanical model of the user's ear. For example, the acoustic input impedance may be used as input data to perform a numerical optimization method, such as non-linear least-squares parameter fit to determine (e.g., one or more parameters of) the equivalent model of the user's ear. In one aspect, the model parameter estimator may minimize the difference between the measured acoustic input impedance and an impedance resulting from the equivalent acousto-mechanical model (e.g., whose parameters are altered), e.g., using the numerical optimization method described herein. In another aspect, the estimator may apply the acoustic impedance into a parameter model that is configured to produce one or more parameters based on the impedance. In another aspect, the estimator may use the impedance to perform a table lookup into a data structure (e.g., stored within memory of the controller 20) that associates one or more parameters of the equivalent model to one or more measured acoustic impedances. Thus, the estimator may compare the measured impedances within the data structure and select parameters that are associated with a measured acoustic impedance that matches the estimated impedance of the user's ear. In one aspect, the determined parameters may be characteristics (e.g., values) of components (e.g., resistor value, capacitor value, etc.) within the equivalent model.

In another aspect, the model parameter estimator 26 may be configured to determine (or estimate) one or more other parameters. For instance, the estimator may determine a parameter associated with an acoustic seal that is created by the output device while it is inserted into (or on) the user's ear. Specifically, the parameter may indicate the effectiveness (e.g., as a numerical value between 0 and 1) of the seal (e.g., at preventing acoustic leakage).

The ear characteristic tracking and decision making system 27 is configured to receive the one or more estimated parameters (e.g., model component values) from the model estimator 26, and is configured to determine whether one or more hearing elements of the user's ear has one or more characteristics based on the received parameter(s). Specifically, the system may receive an equivalent acousto-mechanical model of (e.g., that is specific for) the user's ear from the model estimator 26, and may use the model to determine one or more characteristics of the user's ear. For instance, the system may compare the received equivalent model to a predefined equivalent model (which may be stored in memory). In one aspect, the predefined model may be a "normal" model of a person's ear, whereby the person's ear is a healthy ear (e.g., having no characteristics (or having characteristics less than a threshold) associated with hearing elements of the person's ear). In other words, the parameters of the normal model are associated with hearing elements that do not have any (e.g., known) characteristics associated with them. In one aspect, the predefined model may be a generic model (e.g., a model that is averaged across one or more other models). In another aspect, the predefined model may be a (e.g., predetermined) model of the user's ear (e.g., that may have been determined once upon a first power up cycle of the output device, and while the device was inserted into (or on) the user's ear). The system determines whether there are any differences between the estimated equivalent model and the predefined model in order to identify whether the user's ear has any characteristics or issues. For instance, the system may compare one or more specific parameters of the equivalent model with corresponding parameters of the predefined model, and based on differences between the parameters may identify one or more characteristics with one or more hearing elements (e.g., which may be associated with the parameters). For example, as described herein, the parameter of the equivalent model associated with stiffness of a stapes may be (at least) a capacitor. Thus, the system may compare a capacitive value of the capacitor of the equivalent model with respect to a predefined capacitive value from the normal model. If the capacitive value is different than the predefined value (e.g., greater, or lesser than a predefined threshold), the system may determine that there may be an issue (as a characteristic) with (at least) the (e.g., stapes of the) user's middle ear.

In one aspect, the system may determine one or more characteristics based on whether the estimated model (or one or more estimated parameters) has drifted over a period of time. Specifically, the system may track an estimated parameter to determine whether the parameter increases (e.g., at a particular rate) over a period of time. If so, the system may determine that there is a characteristic of a hearing element that is associated with such drift.

In one aspect, the system may perform one or more operations upon determining that there are one or more characteristics with one or more hearing elements of the user's ear. In particular, the system may transmit a notification to the user indicating that there may be a possible issue with the user's ear. For example, the output device may use a notification audio signal that includes the notification to drive the speaker 22 in order to notify the user. In another aspect, the system may present a (e.g., pop-up) notification on a display screen that is communicatively coupled with the output device, such as a display screen of the source device 2. In one aspect, in addition to (or in lieu of) notifying the user of a detected characteristic, the notification may provide a suggestion to the user to visit a medical provider (e.g., an audiologist) who may exam the user's ear in order to provide a medical diagnosis. In another aspect, the system may transmit the notification to another electronic device, which may present (or keep track) of the determined one or more characteristics. In some aspects, the system may transmit the notification to one or more application programs that are being executed by one or more electronic devices (e.g., on the output device), which may keep track and/or output an alert regarding the characteristics. Thus, the system is able to provide a notification to the user in order to seek a medical provider in order for the user to obtain a diagnosis from the medical provider, which may improve hearing health of the user.

In one aspect, the system may track determined characteristics of hearing elements over a period of time, and may be configured to provide the notification to the user based changes to the characteristics. For example, the system may determine whether the characteristic persists over the period of time (e.g., a determined characteristic being detected a threshold amount over the period of time), if so, the system may provide the notification indicating that the characteristic persists.

In some aspects, the system 27 may determine and track ear characteristics based on a machine learning (ML) algorithm that is performed by the controller 20. For example, the system may use the ML algorithm to determine, having one or more estimated model parameters as input into the algorithm, one or more characteristics of one or more hearing elements of the users as output. In some aspects, the system may use any type of ML algorithm to perform this determination (e.g., a deep neural network (DNN)), etc.).

In another aspect, to track characteristics, the system may track the changes of one or more parameters. For example, the parameters may change based on changes to input impedance over a period of time. As described herein, a determined characteristic may be based on whether an estimated parameter exceeds (or drops below) a predefined parameter of a predefined model by a threshold. The system may track the differences between estimated parameters and predefined parameters for the period of time. For instance, the system may track a rate at which a parameter changes (e.g., based on changes to the input impedance) with respect to the predefined parameter, over the period of time. Upon determining that the rate of change exceeds a threshold, the system may notify the user.

In another aspect, the system may notify the user of a severity level (or value) of a determined characteristic based on the rate of change (and/or based on differences between one or more estimated parameters and predefined parameters). For example, upon determining that a parameter has a particular rate of change, the system may notify the user of the characteristic and a severity level of the characteristic (e.g., low, medium, high). In particular, the system may determine whether the determined rate of change is greater than a first (e.g., low) threshold and/or whether the determined rate of change is greater than a second (e.g., high) threshold (that is greater than the first threshold). If below the first threshold, the severity level may be low, if above the first threshold but below the second threshold, the severity level may be medium, and if above the second threshold, the severity level may be high. More about severity levels is described herein.

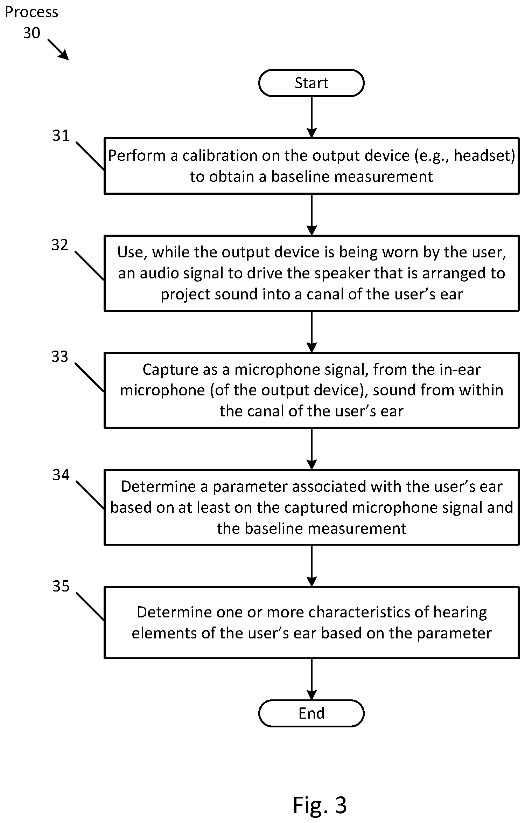

**Fig. 3** is a flowchart of one aspect of a process 30 for determining characteristics of a user's ear. In one aspect, the process 30 may be performed by (e.g., the controller 20 of) the output device (e.g., headset) 3. Specifically, at least some of the operations described herein may be performed by at least some of the operational blocks described in **Fig. 2****.** In one aspect, the process 30 may be performed while the output device plays back audio content (e.g., by using the input audio signal to drive the speaker 22).

The process 30 begins by the controller 20 performing a calibration of the headset to obtain a baseline measurement (at block 31). Specifically, the controller may determine one or more impedance calibration parameters for an impedance model. For example, the controller may receive one or more impedance calibration parameters 28, which may have been previously determined by an off-line impedance calibration (e.g., in a controlled environment), as described herein. In another aspect, the obtained parameters may be based on an on-line impedance calibration, wherein the impedance calibrator 25 may perform an impedance measurement in a holding case of the output device, and may determine the parameters based on the measurement. The controller 20 uses, while the output device is being worn by a user, an audio signal to drive a speaker that is arranged to project sound into the user's ear canal (at block 32). The controller captures as a microphone signal, from the in-ear microphone 21 of the output device, sound from within the user's ear canal (at block 33). The controller determines a parameter associated with the user's ear based at least on the captured microphone signal and the baseline measurement (at block 34). Specifically, the parameter may be a parameter of an equivalent acousto-mechanical model that is equivalent to the user's physical ear. For example, the controller may use the microphone signal to determine a (e.g., S-path) transfer function that represents a response between the speaker and the in-ear microphone, where the determined parameter is based on the S-path transfer function. In particular, the controller may estimate (or measure), using the S-path transfer function, the acoustic input impedance of the user's ear canal with respect to the baseline measurement (e.g., the impedance calibration parameters 28). In particular, the acoustic input impedance may be estimated by applying the determined one or more impedance calibration parameters and the S-path transfer function to the impedance model (as described herein). In one aspect, the estimated impedance may be used to determine the (one or more) parameters that are associated with the user's ear.

The controller 20 determines one or more characteristics of hearing elements of the user's ear based on the determined parameter (at block 35). Specifically, as described herein, the system 27 may compare the determined parameter with predefined parameters, and based on differences (or changes) between the parameters may identify one or more characteristics with hearing elements (e.g., the middle ear, the inner ear, etc.) of the user's ear. In one aspect, the controller transmits a notification related to the one or more characteristics of the one or more hearing elements of the user's ear based on the parameter. In particular, the controller determines the one or more characteristics based on the parameter, as described herein, and transmits the notification, which may include an indication of the characteristic. As described herein, the notification may be an audible notification and/or a visual notification. In some aspects, the notification may be transmitted upon a determination of the one or more characteristics, as described herein.

Some aspects perform variations of the process 30. For example, the specific operations of at least some of the processes may not be performed in the exact order shown and described. The specific operations may not be performed in one continuous series of operations and different specific operations may be performed in different aspects. For example, the controller may perform the calibration before a remainder of the operations of the process 30 are performed. Specifically, the output device may perform the calibration before performing the operations in blocks 32-35. In another aspect, the calibration may be performed while the device is in a controlled environment, as described herein. In which case, the controller may use a signal (e.g., separate from the input signal used to drive the speaker in block 32) to drive the speaker and capture a microphone signal from the in-ear microphone that includes sound produced by the speaker. The controller may use the signals to estimate a response at the in-ear microphone as the baseline measurement, which may be used to determine the parameter, as described herein.

In another aspect, the controller 20 may determine one or more parameters based on one or more estimated impedances. As described herein, the controller may use an audio signal to drive the speaker to project sound into the user's ear canal. In one aspect, the sound may be output at a loudness level. For example, the loudness level may be user defined and/or may be a predefined level. In one aspect, the controller may determine multiple impedances, where each impedance is determined while the audio output device is (e.g., driving the speaker with a same or different audio signal) at a different loudness level. For instance, the controller may determine a first impedance based on a first audio signal that is output at a first loudness level and may determine a second impedance based on a second audio signal that is output at a second (e.g., different) loudness level. Thus, the controller may observe changes in the estimated impedance at different loudness levels and may determine one or more parameters based on impedance change. Thus, the controller is configured to measure the acoustic impedance at two or more different sound levels to detect a non-linear response of the user's ear (e.g., the middle and/or inner ear).

In one aspect, the operations for measuring and tracking ear characteristics may be performed by an output device that is inserted (or covering) one or more ears of the user. Specifically, these operations may be performed by a headset device that is configured to perform audio signal processing operations and/or networking operations in order to stream audio content for playback to the user. In one aspect, these operations may be performed by a headset that may seal off the user's ear (ear canal) from the ambient environment. For example, the headset may be in-ear headphones that include ear tips that are used to create a seal within at least a portion of the user's ear canal when inserted. In one aspect, these operations may optimally be performed by a sealing-type device that prevents air leaking into (and out of) the user's ear canal. Thus, the operations to measure and track ear characteristics may be performed by any (e.g., consumer) electronic head-worn device, rather than being a specialized measurement tool.

In one aspect, the controller 20 may be configured to perform one or more audio signal processing operations based on elements that are coupled to the controller. For example, the controller 20 may include a sound-pickup beamformer that can be configured to process the audio (or microphone) signals produced two or more external (and/or two or more in-ear) microphones of the output device to form directional beam patterns (as one or more audio signals) for spatially selective sound pickup in certain directions, so as to be more sensitive to one or more sound source locations. In some aspects, the controller may perform audio processing operations upon the audio signals that contain the directional beam patterns (e.g., perform spectrally shaping).

In one aspect, the controller may optionally perform additional DSP operations. For example, the controller may apply one or more scalar gains to the input audio signal and/or may spectrally shape the signal by applying one or more filters (e.g., a low-pass filter, a band-pass filter, a high-pass filter, etc.). In another aspect, the controller may perform spatially rendering operations upon one or more of the signals (and/or the mix), by applying spatial filters, such as head-related transfer functions (HRTFs) to produce binaural audio signals for driving one or more speakers (e.g., a left speaker and a right speaker), as described herein. As another example, when the output device includes one or more loudspeakers, the controller may render a HOA representation of the audio signal(s) and downlink signal to produce one or more loudspeaker driver signals (e.g., based on a predefined loudspeaker configuration).

In one aspect, operations performed by the controllers may be implemented in software (e.g., as instructions stored in memory and executed by either controller) and/or may be implemented by hardware logic structures as described herein.

As described herein, the controller 20 may be configured to measure and track ear characteristics of one or more hearing elements of a user's ear. In one aspect, a characteristic may include an obstruction or blockage within a user's ear canal. Such a blockage may be the result of a foreign object, or of a natural accumulation of cerumen (or earwax) that is produced by a person's body to protect the ears. Although a small blockage may be unnoticeable to a person, a large blockage (e.g., a large buildup of cerumen) may have an adverse effect on a person's hearing, as well as cause other health issues (e.g., ear pain, dizziness, etc.). Detecting and tracking these issues may help a person effectively manage characteristics, such as ear canal blockages, (e.g., by notifying the user of characteristics in order for the person to schedule a consultation with a medical provider) thereby improving the person's overall ear health.

The present disclosure provides the ear characteristic tracking and decision making system that is configured to identify ear characteristics, as well as track such characteristics over a period of time. Specifically, to detect and track characteristics such as blockages, the controller is configured to produce a set of one or more reflection parameters of a user's ear canal, using one or more microphone signals that include sound produced by one or more speakers and reflections of the sound (e.g., off of the user's ear canal, ear drum, etc.). The reflection parameters may indicate (or represent) a reflection pattern of the user's ear canal, in response to an audible stimulus. For example, when speaker output travels through an ear canal to the ear drum, it creates resonances and reflections on the way. Most of the sound energy may propagate further to the ear drum through an ear canal with less (or little) blockages (or objects) than an ear canal that includes more (or more sever) blockages. As a result, an ear canal with little blockages may produce a specific reflection pattern that is captured by the one or more microphones, whereas an ear canal with more (or more sever) blockages may produce a different reflection pattern. Using the reflection parameters, the controller may filter microphone signals, and may use the filtered (or output) signals to determine whether a blockage has occurred and/or determine a severity level of a blockage over a period of time. Thus, the system may track a severity level of identified characteristic in order to provide the user with notifications of the progression of severity of characteristics. Knowing the severity of a particular characteristic may provide additional information that may be used by the user for aiding the user in determining whether to seek advice from a medical provider (e.g., an audiologist).

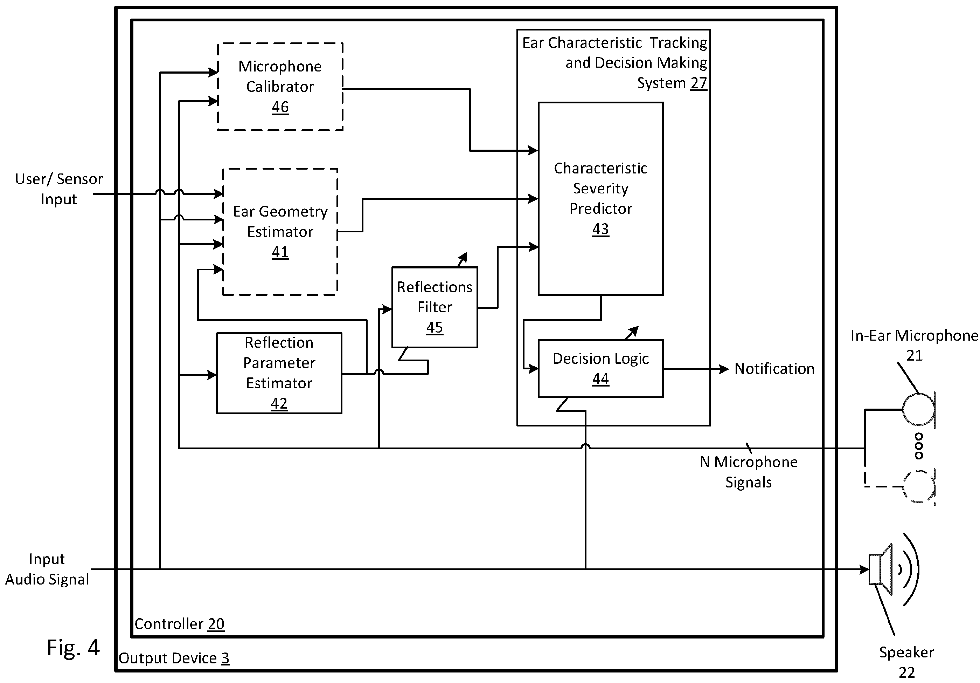

**Fig. 4** shows another block diagram of the output device that is configured to determine a characteristic of the user's ear based on reflection parameters of the user's ear canal from one or more microphone signals according to one aspect. In another aspect, the output device may also be configured to determine a severity of the determined characteristic, as described herein. As shown, the output device includes the controller 20, the speaker 22, and one or more in-ear microphones 21. In one aspect, the output device may include more or less elements. For example, the output device may include an array of in-ear microphones that include two or more in-ear microphones from which the controller 20 may receive two or more (or N) microphone signals

As shown, the controller 20 includes several operational blocks, such as a reflection parameter estimator 42, a reflections filter 45, an ear geometry estimator 41, a microphone calibrator 46, and the ear characteristic tracking and decision making system (or system) 27. In one aspect, the controller may include more or less operational blocks, such as having one or more operational blocks, such as the microphone calibrator and the ear geometry estimator (which are illustrated as having dashed boundaries).

As described herein, the controller may perform one or more audio signal processing operations, while the output device is in use by the user. Specifically, the output device may be worn by (or on) the user, while the controller is performing one or more operations, as described herein. For examples, similar to **Fig. 2****,** the output device may be a headset, such as an earbud that is inserted into the ear canal 6 of the user's ear 5, while the device is in operation. In another aspect, the controller may be using the input audio signal to drive the speaker 22, while the output device is being worn by the user. In addition, the N in-ear microphone(s) 21, where N is the number of in-ear microphones of the output device, may (each) be capturing sound from within the canal of the user's ear. Thus, the controller 20 may be configured to obtain N microphone signal(s), at least one from each microphone, that each include the captured sound.

The reflection parameter estimator 42 is configured to receive the N microphone signals captured by the in-ear microphones, and is configured to use the signals to estimate (or generate) a set of one or more reflection parameters of the user's ear canal. In one aspect, the reflection parameters may be based on a frequency band (e.g., sub-band) analysis of spectral content within one or more of the microphone signals. Specifically, the reflection estimator may compare, for each frequency band of several frequency bands that span a frequency range (e.g., frequency range that is audible to a person), spectral content of the microphone signals to determine whether the spectral content includes reflections of sound produced by the speaker. These reflections may be sound that reflects off of hearing elements of the user's ear (e.g., sound that reflects off of the user's ear canal, the ear drum, etc.). In another aspect, the estimator is determining whether each frequency band primarily includes sound produced by the speaker (e.g., direct sound from the speaker) or the reflections off of hearing elements. In one aspect, the estimator determines, over a period of time (e.g., a second, a minute, an hour, etc.), whether the spectral content across a frequency band includes a threshold number of reflections of the sound produced by the speaker. If so, it may be determined that the frequency band primarily includes reflections. Thus, it may be determined that the spectral content across a frequency band primarily includes reflections based on a number of reflections that are detected within that band. In another aspect, the spectral content may also include (or at least partially include) the direct sound produced by the speaker.

In one aspect, the reflection parameters may be produced by calculating a spatial correlation matrix between one or more microphone signals, where frequency bands

(or sub-bands) between the microphone signals that have spectral content above a threshold (e.g., having an energy level above an energy threshold level), may be determined to indicate a reflection in that frequency band. In one aspect, the number of reflections within a frequency band may be identified by tracking spectral content within the band over a period of time (e.g., tracking how many times spectral content within a sub-band exceeds the threshold over a period of time). In one aspect, a high number of reflections (e.g., above a threshold, above an average, etc.) and/or having a high energy level may indicate reflections that are coming from a location other than the ear canal (e.g., reflections that are due to sound reflecting off of at least a portion of the output device that is inserted into (or placed on) the user's ear canal).

In one aspect, a reflection parameter may indicate whether at least a portion of a microphone signal is to be filtered (e.g., removed) out (e.g., when determining whether there is a characteristic with the user's ear). For instance, the reflection parameter may be a binary value, which indicates whether spectral content across a respective frequency band has (or has been determined to have) reflections of the user's ear canal. For example, when the parameter is at a high value ("1"), the spectral content may be associated with (e.g., primarily) reflections, while when the parameter is a low value (e.g., "0"), the spectral content does not primarily have reflections (or may include reflections that are below a threshold).

In one aspect, the reflection parameter estimator may use estimated reflection parameters to produce a reflection pattern (e.g., a frequency band reflection map). Specifically, the estimator may determine the reflection pattern based on an effective rank estimation of reflections (or the reflection parameters) within one or more frequency bands. Thus, the pattern may indicate whether (or which of) one or more frequency bands includes a high number (e.g., above the reflections) of reflections (e.g., across a frequency range).

In another aspect, one or more reflection parameters may be produced by looking at one microphone signal in a similar way, as described herein. For instance, the reflection estimator may determine a reflection parameter across one or more frequency bands, using the one microphone signal (e.g., determining that a reflection is present in a frequency band based on spectral content within that band being above a threshold).

In one aspect, the reflection estimator 42 may estimate the reflection parameters (and/or a reflection pattern) based on a MLML algorithm. Specifically, the estimator may include the ML algorithm that receives the one or more in-ear microphone signals as input and may produce (or predict) a number of (sub-band) reflections associated with the one or more microphone signals. Based on the number, the reflection estimator may generate one or more reflection parameters. In one aspect, the reflection estimator may estimate a parameter for each frequency band of several frequency bands that span a frequency range (e.g., an audible frequency range for a person). In another aspect, any method may be used to determine the reflection parameters of the user's ear canal based on one or more microphone signals.

In one aspect, in addition to (or in lieu of) determining whether reflections are being received (or detected) in one or more microphone signals, the reflection parameter estimator may be configured to determine (or estimate) a direction of arrival (DOA) of the received reflections. Specifically, the estimator may be configured to track reflections that originate from a particular region within the user's ear (e.g., reflections that occur inside or from the ear canal), as opposed to reflections that do not originate from inside the ear canal, such as direct sound (or other reflections) that may occur elsewhere (e.g., based on a direct energy path produced by the loudspeaker as it projects sound into the ear canal). In one aspect, these other reflections may be produced as a result of sound reflecting off a portion (e.g., a front portion) of the headset, such as reflecting off an ear tip of the headset that is inserted into and creates a seal in the user's ear. Thus, the reflection parameter estimator may be configured to use the one or more microphone signals to produce a directional beam pattern that is directed into (or toward) the canal of the user's ear and away from the output device. Specifically, estimator may estimate a frequency band direction of arrival (DOA) parameter by analyzing one or more frequency bands that include a strongest signal (e.g., having a highest spectral content with respect to spectral content of other frequency bands). In one aspect, these frequency bands (e.g., with the strongest signal) may represent a direct sound path between the loudspeaker and the one or more microphones. Thus, the estimator may estimate a DOA parameter for each frequency band that is associated with reflections from within the ear canal (e.g., within a particular region towards which the beam pattern is directed). In one aspect, the parameter estimator may produce a DOA mask that includes each of the (e.g., DOA parameters for each) frequency band at which reflections originate from within the ear canal (e.g., thereby filtering out frequency bands associated with the direct path between the speaker and the microphone(s), and other reflections that do not originate from the region of interest).

In one aspect, the reflection parameter estimator 42 is configured to combine (or add) the DOA parameters to the reflection parameters. In one aspect, the parameters may be combined by multiplying the parameters in the frequency domain. In another aspect, the parameter estimator may combine the reflection pattern of one or more reflection patterns and the DOA mask of one or more DOA parameters to create a reflection mask. In one aspect, the one or more estimated reflection parameters may define the reflection mask. More about filtering signals is described herein.

In one aspect, the reflections filter 45 is configured to receive the estimated reflection parameters from the reflection parameter estimator 42 and receive (or obtain) the N microphone signals, and is configured to filter the N microphone signals with the reflection parameters to produce one or more output signals. Specifically, the reflection filter may be defined (or generated) using the one or more estimated reflection parameters to filter spectral content (e.g., in the frequency domain) out of the microphone signals that is not associated with the reflection parameters. In particular, the reflection filter may remove spectral content associated with reflection parameters that have a low value, thereby indicating that the spectral content does not (e.g., primarily) include reflections. In one aspect, the reflection filter may be defined using the created reflection mask, which may be a binary mask in which reflections of microphone signals may be filtered out (e.g., as output signals) when processed using the mask. For example, the output (filtered) signals may include energy only coming from reflections of the speaker sound output that have been reflected back from the inside of the ear canal and have been received by the in-ear microphone(s). Reflections that may originate from regions other than the ear canal and/or direct sound energy that originates from the speaker may be filtered out by the reflection mask. In one aspect, the reflections filter 45 may produce a same number of filtered signals (N signals), as the number of microphone signals.

In one aspect, the microphone signals that are filtered by the reflections filter may be obtained after the microphone signals that were used to generate the one or more reflection parameters. For example, the reflection parameters may be estimated over a period of time (e.g., an hour, a day, etc.). Once estimated, the reflection filter 45 may receive the reflection parameters and may obtain microphone signals that are obtained after the period of time in order to filter the subsequent microphone signals.

In one aspect, the reflections filter 45 may be dynamically updated (or changed) based on changes to reflections within the user's ear canal. For example, the reflection parameter estimator may be configured to adjust reflection parameters based on changes to spectral content captured within the one or more microphone signals. For example, the reflection parameter estimator may monitor one or more frequency bands to determine whether reflections in those bands exceed the threshold, as described above. Upon determining that that the number of reflections for a frequency band have decreased (e.g., from a previous time at which a reflection parameter was estimated), the parameter estimator may adjust the parameter (e.g., from a high value to a low value). As a result, the reflections filter may be dynamically updated with changing reflection parameters.

In one aspect, the ear geometry estimator 41 is configured to estimate the geometry (e.g., size, shape, length, etc.) of the user's ear (e.g., ear canal). In one aspect, the geometry estimator may be configured to receive the one or more reflection parameters (e.g., or reflection mask) estimated by the reflection parameter estimator 42, and may be configured to estimate the ear geometry based on the parameters. For instance, the geometry estimator may be configured to perform a table lookup into a data structure (e.g., stored within the controller 20 of the output device 3) that associates one or more ear geometries with one or more (e.g., predefined) reflection parameters (or masks).

In another aspect, the ear geometry may be estimated through other means. For example, the geometry estimator may be configured to receive the N microphone signals and receive the input audio signal that is being used to drive the speaker, and may be configured to determine the geometry based on the signals. For instance, the geometry may use the signals to determine a (e.g., impulse) response at one or more microphones, and may determine the geometry based on the response. In another aspect, the geometry estimator may determine the geometry based on just the microphone signals (e.g., without the input audio signal).

In another aspect, the ear geometry may receive user input and/or sensor input, and may determine the ear geometry based on the input. As described herein, the output device may be a pair of earbuds, where each earbud has an ear tip that is used to create a seal within the user's ear canal when the earbuds are inserted into the user's ear. In one aspect, the output device may be fitted with one or more different types or sizes of ear tips. For example, the user may couple a particular ear tip (e.g., an ear tip with a small diameter with respect to other ear tips) to the output device that is sized to create a seal within the user's ear canal. For example, the user may fit a large sized ear tip to the output device when a smaller sized ear tip is not large enough (e.g., not having a large enough diameter) to create the seal, which may be due to the size of the user's ear canal. In one aspect, the ear geometry estimator may receive user input that indicates what type (e.g., size, such as small, medium, or large) of ear tip is coupled to the output device. From the type, the ear geometry may determine the geometry (e.g., size) of the ear canal. Returning to the previous example, when the user indicates that large sized ear tips are coupled to the output device, the geometry estimator may determine that the ear canal is large (e.g., bigger than average). In another aspect, the ear geometry estimator may determine the geometry of the user's ear based on sensor input from one or more sensors. For example, the ear geometry estimator may receive image data captured by one or more cameras that are communicatively coupled with the controller. From the image data, which may include an image of the user's ear (and/or ear canal), the estimator 41 may determine the geometry of the user's ear. In another aspect, the estimator 41 may use any method to determine the geometry of the user's ear. In another aspect, the ear geometry estimator may use a combination of the one or more methods described herein to determine the geometry.

The characteristic severity predictor 43 is configured to receive the output (or filtered) signals from the reflection filter 45, and is configured to determine one or more characteristics of a hearing element (e.g., determine whether there is a blockage within the ear canal) based on the output signals. In one aspect, the predictor may be a ML algorithm (e.g., a deep neural network (DNN)) that is configured to take the filtered signals as input, and determine whether one or more hearing elements has one or more characteristics. For example, the DNN may be configured to output an indication that there is a blockage within the ear canal based on the filtered signals. In one aspect, the predictor may be configured to determine one or more physical characteristics of the blockage. For example, the predictor may determine an amount (e.g., size, shape, length, etc.) of the blockage and/or the type of blockage (e.g., whether the blockage is cerumen, whether the blockage is a fluid, etc.).

In one aspect, the predictor may be configured to receive the estimate of the ear geometry, and may be configured to determine a characteristic based on the ear geometry. For example, the predictor may determine whether there is a blockage within the ear canal based on a comparison between the ear geometry and the filtered signals (and/or between the estimated ear geometry and the determined physical characteristics of the predicted blockage). Specifically, the predictor may determine whether the predicted blockage (or at least a portion of the blockage) is actually a part of the natural shape, size, etc., of the user's ear canal. For instance, characteristics of ear canals may differ between people, such as one person may have a narrow ear canal, while another person has a wider ear canal. In which case, the predictor may determine whether there is blockage based on differences between the estimated ear geometry of the user and the physical characteristics of the predicted blockage. For instance, the ear geometry may indicate that (at least a portion) of the user's ear canal is tubular shaped and having a particular diameter. The predicted blockage, however, may be similarly shaped along the same portion, but having a narrower diameter. The blockage may have a narrower diameter due to an object within the ear canal that reduces the diameter through which air (and sound) may travel to the user's ear drum. As a result, the predictor may determine that the blockage is sized based on the difference between the smaller diameter of the blockage and the larger diameter of the user's (natural) ear geometry.

In another aspect, the predictor 43 may be configured to receive a calibration of the in-ear microphones 21 from the microphone calibrator 46, and may be configured to predict the one or more characteristics based on the calibration and the filtered signals. In one aspect, the calibrator may be configured to receive the input audio signal and the microphone signal from the in-ear microphone, and may be configured to perform a calibration of the microphone (e.g., based on an impulse response measurement of output sound of the speaker at a location of the microphone). In one aspect, the calibration operation may be performed prior to (or after) the output device is worn by the user. For instance, the predictor 43 may use the calibration to take into account characteristics of the microphones (e.g., such as age, etc.), and from the calibration process the filtered signals to compensate for the microphone characteristics. In another aspect, the calibration operations performed by the calibrator may be the same (or similar) as the impedance calibration performed by the impedance calibrator 25. In another aspect, the microphone calibrator may use any calibration method to compensate for microphone characteristics.

In one aspect, the operations of the ear geometry estimator 41 and the microphone calibrator 46 are optional (as indicated by these boxes being dashed lines). For example, the characteristic severity predictor may predict whether there is a characteristic based on the filtered signals. In another aspect, the predictor may receive data from one or both of these blocks based on components that are communicatively coupled to the controller. As described herein, the output device may include N in-ear microphones 21, where the controller receives N microphone signals. In one aspect, the predictor may obtain the calibration from the microphone calibrator when the output device only includes one microphone. Or more specifically, the predictor may use the calibration when the operations described herein are performed when only one microphone signal is obtained (e.g., and used to estimate the reflection parameters and is filtered using the parameters). In another aspect, the predictor 43 may not obtain the calibration (e.g., and the operations of the microphone calibrator may not be performed), when the N microphone signals includes two or more microphone signals that are obtained by N in-ear microphones of the output device.

In one aspect, the characteristic severity predictor 43 is configured to determine a severity level of the predicted characteristic. Specifically, the severity level may be a numerical value (e.g., between 0 - 1), where a low value (e.g., 0.1) indicates a low severity, while a high value (e.g., 1) indicates a higher severity than the low severity. For example, with respect to a blockage, the severity level may indicate one or more characteristics of the blockage, such as size, shape, length, etc., where a low severity level indicates that the blockage is small (e.g., with respect to the size of the ear canal). A high severity level, however, may indicate the blockage is more severe than that of a lower severity level (e.g., the blockage being larger with respect to the ear canal). In one aspect, the severity level may correspond to an amount of open space within the ear canal. For example, a blockage having a low severity level may represent a blockage where the diameter of open space within the ear canal is above a threshold. Conversely, a blockage may have a high severity level when the diameter of open space within the ear canal is below the threshold.

In one aspect, the predictor may use the estimate of the ear geometry to determine the severity level, as described herein. For example, the predictor may determine a severity level of the blockage based on a comparison of the ear geometry and the filtered signals (and/or characteristics of the ear canal that are determined by the predictor based on the filtered signals). As an example, the ear geometry may indicate that the ear canal is of a particular diameter, and the filtered signals may indicate a blockage that reduces the diameter. Thus, the predictor may determine the severity based on the reduction (or difference) of the diameter caused by the blockage.

In another aspect, the predictor may determine a severity based on a comparison of a severity value with one or more thresholds. For example, the predictor may determine a severity value based on the filtered signals. As described herein, the severity value may be a value between 0 to 1. Using the severity value, the predictor may determine a severity level of the blockage based on whether the severity value is below one or more thresholds. As described herein, one or more thresholds may indicate a severity level, such as low, medium, and/or high. In which case, when the severity level is below a low threshold and a high threshold (that is higher than the low threshold), the severity level may be low. If the severity value is higher than the low threshold but lower than the high threshold, the severity level may be medium. And when the severity value is above the high threshold, the severity level may be high.

The decision logic 44 is configured to receive the determined characteristic and/or the predicted severity level of the characteristic, and is configured to determine whether the predictor's assessment is accurate. Specifically, the decision logic is configured to determine a confidence score of the predictions made by the predictor, and determine whether the confidence score exceeds a threshold. For instance, the decision logic is configured to receive the input audio signal that is used to drive the speaker 22, and is configured to determine a confidence score based on the audio content contained within the input audio signal. In one aspect, the determined confidence score may be based on the type of audio content within the input audio signal. For instance, the confidence score may be low when the audio signal includes (e.g., primarily) speech. Conversely, the confidence score may be high (or higher than a threshold) when the audio signal includes a musical composition. In one aspect, the confidence score may be based on characteristics of the audio content, such as whether or not the audio content includes transitions or decays. For instance, when the audio content includes one or more decays (e.g., a transition when sound fades from one intensity to another intensity (e.g., to silence)), the decision logic may determine that a high confidence score. As another example, when the audio content does not include decays (or includes a number of decays below a threshold), such as when the audio content is constant (e.g., having a constant signal level, or a signal level that does not go below a threshold), the confidence score may be low. In another aspect, the confidence score may be based on a playback level of the input audio signal (e.g., when the playback level is above a threshold, the confidence score may be low). In another aspect, the confidence score may be based on other characteristics of the input audio signal.

The decision logic is configured to determine whether the determined confidence score exceeds a (e.g., predefined) threshold. If so, the decision logic may be configured to output a notification regarding the determined characteristic. For example, the logic may output (e.g., an audible) notification indicating that there is a blockage within the ear canal and/or may indicate a severity of the blockage. In which case, when the severity level is low, the notification may indicate that the user may have a small blockage within the ear canal. In another aspect, the notification may provide a suggestion for the user to seek a medical opinion regarding the determined characteristic.

As described herein, the ear characteristic tracking and decision making system 27 may be configured to track one or more characteristics of one or more hearing elements the user's ear. In one aspect, the system may track predicted blockages within the user's ear canal. For instance, upon determining that there is a blockage, the system may track the blockage for a period of time (e.g., a day, a week, a month). In particular, the system may perform one or more of the operations described herein in order to predict whether there is a blockage and the severity of the block one or more times over the period of time. In one aspect, the system may output a notification in response to determining that a severity level of the blockage is increasing (e.g., the severity value increasing at a predefined rate).

In one aspect, the controller 20 may perform one or more additional audio signal processing operations. For example, the controller may perform echo cancellation operations in order to cancel out all (or most of) the speaker signal that is coming directly from the speaker to the in-ear microphone(s). For example, the controller may determine one or more linear filters based on a transmission path between the in-ear microphone(s) and the speaker, and apply the filter to input audio signal to generate an estimate of echo, which is subtracted from the N microphone signals captured by the in-ear microphone(s). In another aspect, the controller may use any method of echo cancellation.

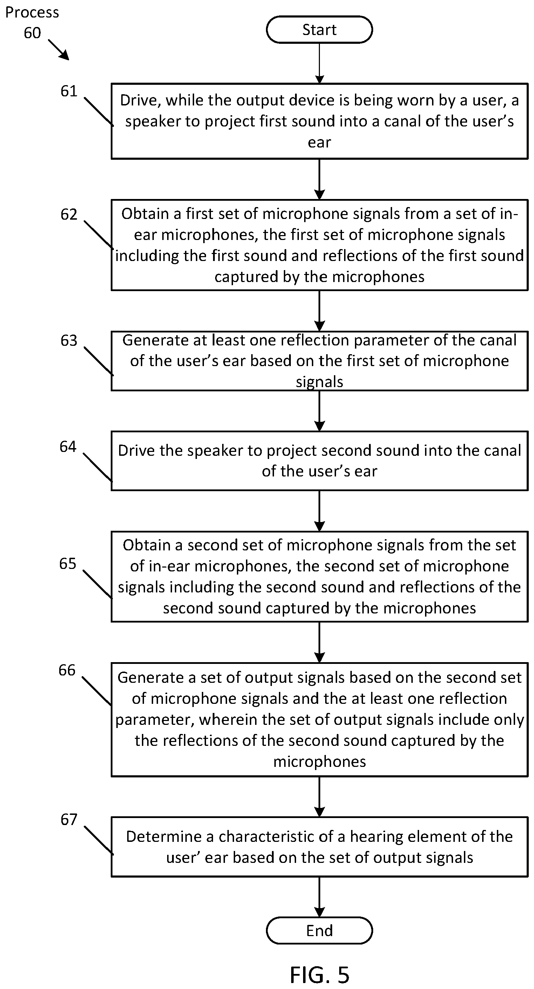

**Fig. 5** is a flowchart of one aspect of a process 60 for determining one or more characteristics of the user's ear based on estimated reflection parameters of the user's ear canal. In one aspect, the process 60 may be performed by (e.g., the controller 20) of the output device 3.

The process 60 begins by the controller 20 driving, while the output device is being worn by the user, speaker 22 to project first sound into a canal of the user's ear (at block 61). Specifically, the controller may use an (e.g., input) audio signal to drive the speaker 22 that is arranged to project sound into an ear canal of the user's ear. As described herein, the audio signal may be a part of a piece of user-desired audio content, such as a musical composition, a podcast, or a movie sound track. The controller obtains a first set of microphone signals from a set of in-ear microphones 21, the first set of microphone signals including the first sound and reflections of the first sound captured by the microphones (at block 62). As described herein, the controller may obtain one or more (or N) microphones signals from one or more microphones of the output device. The microphone signals may include the sound produced by the speaker (e.g., a direct sound path between the speaker and the microphone(s)) and reflections of the sound from within the user's ear canal (e.g., sound that reflects off of the inner wall of the ear canal, reflects off the ear drum, etc.). The controller generates at least one reflection parameter of the canal of the user's ear based on the first set of microphone signals (at block 63). Specifically, the reflection parameter estimator 42 may produce one or more reflection parameters, each indicating a number of reflections that are received by the microphones in one or more frequency bands. In addition, the estimator may estimate DOA parameters based on a directional beam pattern produced by one or more of the first set of microphone signals that is directed towards the canal of the user's ear and away from the output device. As described herein, these parameters may be combined (e.g., to create a reflection mask)

The controller 20 drives the speaker to project second sound into the canal of the user's ear (at block 64). For example, the second sound may be a part of the input audio signal that was used to drive the speaker to project the first sound. The second sound, however, may be associated with a portion of the signal that is after a portion of the signal that is associated with the first sound (e.g., the second sound being played back after the first sound). In another aspect, the second sound may be associated with a different audio signal from the input audio signal that was used to drive the speaker when projecting the first sound. The controller obtains a second set of microphone signals from the set of in-ear microphones, the second set of microphone signals including the second sound and reflections of the second sound captured by the microphones (at block 65). In one aspect, the second set of microphone signals may be captured after the first set of microphones. For instance, once the reflection parameters are estimated, the controller may obtain the second set of microphone signals. In some aspects, the second set of microphone signals may be obtained after a period of time from when the reflection parameters are estimated (e.g., a minute, an hour, a day, etc.).

The controller 20 generates a set of output (e.g., filtered) signals based on the second set of microphone signals and the at least one reflection parameter, where the set of output signals include only the reflections of the second sound captured by the microphones (at block 66). Specifically, the reflections filter 45 uses the one or more reflection parameters (or reflection mask) to filter spectral content from the second set of microphone signals that is not associated with reflections of (or from) the ear canal of the user's ear. For example, the reflections filter uses the reflection parameters to filter the second sound from the second set of microphone signals by removing spectral content across one or more frequency bands that are not associated with the at least one reflection parameter.

The controller 20 determines one or more characteristics of one or more hearing elements of the user's ear based on the set of output signals (at block 67). Specifically, the characteristic severity predictor 43 is configured to receive the output signals and apply the output signals as input into a DNN (or characteristic severity model), which determines one or more characteristics as output of the DNN. For example, the predictor may determine that there is a blockage within the user's ear canal based on the output signals. In another aspect, the predictor may determine (e.g., physical) characteristics of the blockage, as described herein, such as a size, shape, etc. of the blockage.

In one aspect, upon determining a characteristic, the controller may present a notification to the user, alerting the user of the characteristic. Specifically, the decision logic may output a notification (e.g., an audible notification via the speaker 22, a visual (pop-up) notification via a display screen, etc.) alerting the user that a characteristic has been detected, and/ or additional information relating to the characteristic, such as a description of the characteristic and/or a severity of the characteristic. For example, when the characteristic is a blockage within the ear canal, the notification may indicate that there is a blockage with a low severity. With such information, the user may seek a medical professional for a medical diagnosis, by an audiologist, for example.

In one aspect, the notification may be based on whether a confidence score associated with audio content of the first sound (e.g., a first audio signal used to drive the speaker to produce the first sound) exceeds a confidence threshold. As described herein, the confidence score may be based on characteristics of the audio content of the first sound (e.g., whether the sound includes a threshold number of transitions, decays, and/or pauses). In response to a confidence score of the first sound exceeding the confidence threshold, the output device may output (present) a notification regarding the determined characteristic of the hearing element.

Some aspects perform variations of the process 30. For example, the specific operations of at least some of the processes may not be performed in the exact order shown and described. The specific operations may not be performed in one continuous series of operations and different specific operations may be performed in different aspects. For example, the severity predictor may determine a severity of the characteristic, as described herein.

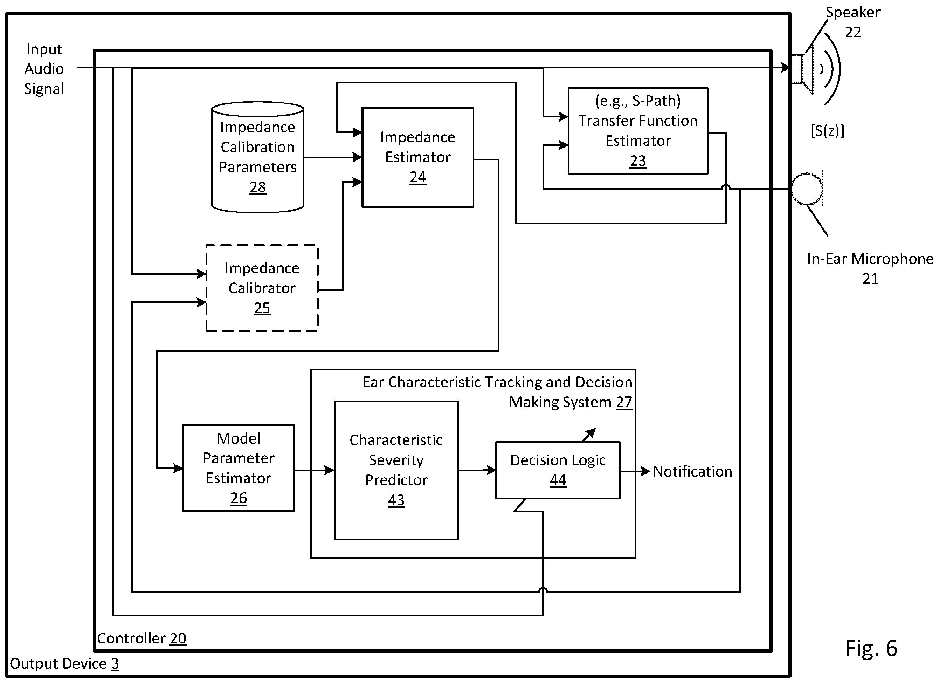

**Fig. 6** shows another block diagram of the output device that is configured to determine a characteristic of a user's ear based on an acoustic input impedance measurement according to one aspect. In one aspect, this diagram shows that the controller 20 includes several operational blocks from **Figs. 2** and **4****,** which may perform ear characteristic tracking and decision making operations, as described herein. In one aspect, the controller may include more or less operational blocks, such as having additional operational blocks as shown in **Figs. 2** and **4****.**

As described herein, this figure shows a diagram in which the controller is configured to determine a characteristic based on an acoustic input impedance measurement. Specifically, this figure illustrates a controller that is configured to determine characteristics and/or predict (determine) characteristic severity (e.g., a severity level of the characteristic) based on an acoustic input impedance measurement using one in-ear microphone. For example, the controller may be configured to determine whether the user's ear canal includes one or more blockages, using the impedance analysis described in **Fig. 2****.** Thus, unlike the controller of **Fig. 4** in which a characteristic, such as blockages, may be predicted based on N microphone signals, which may be two or more microphone signals, this figure illustrates that such predictions may be made based on one microphone signal.

As described herein, the model parameter estimator 26 is configured to receive estimated acoustic input impedance from the impedance estimator 24, and is configured to use the acoustic impute impedance to determine an equivalent acousto-mechanical model of the user's ear. In one aspect, one of the parameters may correspond to (or be equivalent) to the user's ear canal. Such a parameter may indicate characteristics of the canal, such as shape, size, length, consistency, etc.

The characteristic severity predictor 43 is configured to receive the estimated parameters, such as the parameter that is equivalent to the user's ear canal, and determine whether there is a characteristic with the user's ear. Specifically, the characteristic severity predictor may be configured to determine whether there is a blockage within the user's ear canal based on a parameter associated with the user's ear canal. For example, the parameter may indicate that the ear canal is shorter than an average person's ear canal, which may be the result of an object or blockage within the ear canal. As another example, the predictor may perform a table lookup into a data structure that associates ear canal parameters with blockages, and upon identifying a parameter in the data structure associated with the estimated parameter, the predictor may determine that there is a blockage.

In another aspect, the predictor may determine whether there is a blockage based on a monitoring of the ear canal parameter. As described herein, the parameter may indicate characteristics of the ear canal. For example, the predictor may determine the characteristics based on the parameter. In particular, the predictor may perform a table lookup into a data structure that associates one or more characteristics of a user's ear canal with the estimated parameter. In another aspect, the characteristics may be associated (or a part of) the parameter. In one aspect, the predictor may monitor these characteristics over time. Specifically, the model parameter estimator 26 may estimate one or more parameters over that time period, which the predictor 43 may use to accumulate characteristics. With the characteristics, the predictor may determine whether there are changes to the ear canal, such as whether the length of the ear canal is decreasing over time. Such changes may be used by the predictor to determine whether the ear canal has a blockage.

In addition, the predictor may determine a severity of the blockage based on the monitoring of characteristics. Specifically, as the predictor monitors a blockage, characteristics of the blockage may be compared to one or more thresholds. For example, a size of the blockage (e.g., with respect to the size of the ear canal) may be compared to a threshold, which when the size exceeds that threshold the severity level of the blockage may be high (e.g., indicating that the blockage is to a size that is adversely restricting the passage of air between the outer ear and the ear drum). As described herein, the severity level may be determined for a blockage, but the severity level may also be determined for any determined characteristic of one or more hearing elements.

As described thus far, the model parameter estimator 26 may be configured to estimate a parameter associated with the ear canal of a user. In another aspect, the parameter estimator may be configured to estimate a parameter for a specific characteristic of one or more hearing elements. For example, the parameter estimator may estimate a parameter associated with a blockage (or object) within the user's ear (e.g., ear canal). In one aspect, the ear characteristic tracking decision making system 27 may use the parameter to determine one or more characteristics of the blockage, as described herein.

Upon determining the characteristic and/or severity level of the characteristic, the decision logic is configured to determine whether a confidence score associated with the input audio signal is above a threshold, as described herein. If so, the decision logic may present one or more notifications, alerting the user of the characteristic and/or the severity level associated with the characteristic.

As has been described above, in order to perform ear characteristic tracking and decision making operations, one aspect of the present technology contemplates gathering and using available data from legitimate and specific sources. It is contemplated that in some cases, data being gathered may include information or data may be unique to an individual and therefore can be used to identify a specific person. For example, the data can include location-based data, home addresses, online identifiers, demographic data, email addresses, telephone numbers, date of birth, other types of information relating to a user's fitness level or health conditions (e.g., measurements of vitals, information related to medications, information related to exercise), or other personal data (e.g., biometric identifiers).

It is contemplated that the personal data can be beneficial to users in the present technology. For example, based on the consent of a user to determine whether one or more hearing elements of the user's ear has characteristics, health information may be used.

It is contemplated that compliance with well-established privacy practices and/or privacy policies will be required for entities that collect, analyze, disclose, transfer, store, or perform any other usage of personal data. For example, as there are industry or governmental requirements for maintaining the privacy of users, these entities are expected to consistently apply and implement practices in privacy that are generally recognized as meeting or exceeding those requirements.

Any information regarding personal data usage should be prominently provided and users should be able to easily access such information. Updates to information should be made when there are changes to the way that data is collected or used. Collection of users' personal information should be limited to legitimate purposes only. Additionally, consent from the user must be received before collection/sharing occurs unless there are other legitimate basis that are specified in applicable law. Steps for safeguarding and securing access are needed to protect personal data and help to ensure that those with access to the personal data adhere to their privacy policies and procedures. It is contemplated that third party evaluation can be made to certify that entities are adhering to widely accepted privacy policies and practices. In addition, adaptability of policies and practices based on both the types of personal data being collected and/or accessed and also applicable laws and standards, such as, for example, stricter jurisdiction-specific considerations. For instance, in the US, federal and/or state laws (e.g., Health Insurance Portability and Accountability Act (HIPAA)) govern the collection of or access to certain health data. In other countries, health data are subject to different regulations and policies and should be handled accordingly.

It is also contemplated that users may selectively block certain usage, or access to, personal data. In those instances, hardware and/or software components can be employed to block or prevent access to such personal data. For example, such as in the case of ear characteristic tracking and decision making, the present technology can be configured to allow users to select to "opt in" or "opt out" of participation in the collection of personal information data while characteristics of the ear are detected and tracked by the output device or anytime thereafter. In another example, users can select not to have audio data used to perform these operations. Specifically, users can select to limit the amount (and length of time) audio data (or any type of data that) is captured and used to track ear characteristics. In addition to providing "opt in" and "opt out" options, the present disclosure contemplates providing notifications relating to the access or use of personal information. For instance, a user may be notified upon the output device obtaining microphone signals to determine whether hearing elements of the user's ear have characteristics, such as blockages, as described herein. In addition, the user may be reminded again just before personal information data is accessed by the output device.

It is contemplated that minimizing the risks of unintentional or unauthorized access or use will impact the way that personal data is managed and handled. For example, one way to minimize risk is to limit the scope of data collection and delete data when it is no longer required. As another example, when possible (e.g., for particular health applications), the present disclosure can de-identify the data in order to protect a user's privacy. Various methods to de-identify may be used, when possible, for instance, by removing labels or names, controlling how much data to store, or controlling the granularity of the data being stored (e.g., location can be collected at country level rather than at an address level or data can be aggregated across users), and/or other methods can be used (e.g., differential privacy).

Finally, although it is contemplated that personal data may be used to implement the various disclosed embodiments, it is also contemplated that various aspects can be implemented without the need for accessing such personal data. In other words, various aspects of the present disclosure are not rendered inoperable due to missing some or all of the personal data. In one example, content can be provided to users based on aggregated data that is not specific to each user or contain only a minimum amount of personal data. In the latter instance, the users may receive only content being processed on the users' devices or other information not personal to the users.

As previously explained, an aspect of the disclosure may be a non-transitory machine-readable medium (such as microelectronic memory) having stored thereon instructions, which program one or more data processing components (generically referred to here as a "processor") to perform the network operations and audio signal processing operations, as described herein. In other aspects, some of these operations might be performed by specific hardware components that contain hardwired logic. Those operations might alternatively be performed by any combination of programmed data processing components and fixed hardwired circuit components.

While certain aspects have been described and shown in the accompanying drawings, it is to be understood that such aspects are merely illustrative of and not restrictive on the broad disclosure, and that the disclosure is not limited to the specific constructions and arrangements shown and described, since various other modifications may occur to those of ordinary skill in the art. The description is thus to be regarded as illustrative instead of limiting.

In some aspects, this disclosure may include the language, for example, "at least one of [element A] and [element B]." This language may refer to one or more of the elements. For example, "at least one of A and B" may refer to "A," "B," or "A and B." Specifically, "at least one of A and B" may refer to "at least one of A and at least one of B," or "at least of either A or B." In some aspects, this disclosure may include the language, for example, "[element A], [element B], and/or [element C]." This language may refer to either of the elements or any combination thereof. For instance, "A, B, and/or C" may refer to "A," "B," "C," "A and B," "A and C," "B and C," or "A, B, and C."

Exemplary embodiments of the present disclosure are set out in the following items:
1. A method performed by a headset that includes a speaker and an in-ear microphone, the method comprising:
   performing a calibration on the headset to obtain a baseline measurement;
   using, while the headset is being worn by a user, an audio signal to drive the speaker to project sound into a canal of a user's ear;
   capturing as a microphone signal, from the in-ear microphone, sound from within the canal of the user's ear;
   determining a parameter associated with the user's ear based at least on the captured microphone signal and the baseline measurement; and
   transmitting a notification related to one or more characteristics of one or more hearing elements of the user's ear based on the parameter.
2. The method of item 1 further comprising using the microphone signal to determine a secondary path ("S-path") transfer function that represents a response between the speaker and the in-ear microphone, wherein the determined parameter is based on the S-path transfer function.
3. The method of item 2, wherein the S-path transfer function is determined as part of an active-noise cancellation process that is performed by the headset to generate an anti-noise signal for driving the speaker.
4. The method of any of items 2-3, wherein determining the parameter associated with the user's ear comprises:
   measuring, using the S-path transfer function, an acoustic input impedance of the user's ear canal with respect to the baseline measurement; and
   using the acoustic input impedance to determine the parameter that is part of an acousto-mechanical model that is equivalent to at least the user's middle ear and outer ear.
5. The method of item 4, wherein performing the calibration comprises determining one or more impedance calibration parameters for an impedance model, wherein measuring the acoustic input impedance comprises estimating the acoustic input impedance by applying the determined one or more impedance calibration parameters and the S-transfer function to the impedance model.
6. The method of any of items 4-5, wherein the acoustic input impedance is measured over a period of time, wherein the method further comprises determining the one or more characteristics based on a change between the parameter and a corresponding parameter of the acousto-mechanical model over the period of time.
7. The method of any of items 1-6 further comprising:
   comparing the parameter to a corresponding parameter of an acousto-mechanical model; and
   identifying the one or more characteristics based on the comparison of the parameter to the corresponding parameter.
8. The method of any of items 1-7, wherein the calibration is performed while the headset is in a holding case that is arranged to house the headset while it is not worn by the user.
9. A headset, comprising:
   a speaker;
   a microphone;
   a processor; and
   memory having instructions stored therein which when executed by the processor causes the headset to
   perform a calibration on the headset to obtain a baseline measurement,
   use, while the headset is being worn by a user, an audio signal to drive the speaker to project sound into a canal of a user's ear,
   capture as a microphone signal, from the microphone, sound from within the canal of the user's ear,
   determine a parameter associated with the user's ear based at least on the microphone signal and the baseline measurement, and
   transmit a notification related to one or more characteristics of one or more hearing elements of the user's ear based on the parameter.
10. The headset of item 9, wherein the memory has further instructions to use the microphone signal to determine a secondary path ("S-path") transfer function that represents a response between the speaker and the microphone, wherein the parameter is based on the S-path transfer function.
11. The headset of item 10, wherein the S-path transfer function is determined as part of an active-noise cancellation process that is performed by the headset to generate an anti-noise signal for driving the speaker.
12. The headset of any of items 10-11, wherein determining the parameter associated with the user's ear comprises
   measuring, using the S-path transfer function, an acoustic input impedance of the user's ear canal with respect to the baseline measurement; and
   using the acoustic input impedance to determine the parameter that is part of an acousto-mechanical model that is equivalent to at least the user's middle ear and outer ear.
13. The headset of item 12, wherein performing the calibration comprises determining one or more impedance calibration parameters for an impedance model, wherein measuring the acoustic input impedance comprises estimating the acoustic input impedance by applying the determined one or more impedance calibration parameters and the S-transfer function to the impedance model.
14. The headset of any of items 12-13, wherein the acoustic input impedance is measured over a period of time, wherein the memory has further instructions to determine the one or more characteristics based on a change between the parameter and a corresponding parameter of the acousto-mechanical model over the period of time.
15. The headset of any of items 9-14, wherein the memory has further instructions to:
   compare the parameter to a corresponding parameter of a predefined acousto-mechanical model; and
   identify the one or more characteristics based on the comparison of the parameter to the corresponding parameter.
16. The headset of any of items 9-15, wherein the calibration is performed while the headset is in a holding case that is arranged to house the headset while it is not worn by the user.
17. A method performed by a headset that includes a speaker and a set of in-ear microphones, the method comprising:
   driving, while the headset is being worn by a user, the speaker to project a first sound into a canal of a user's ear;
      receiving a first set of microphone signals from the set of in-ear microphones, the first set of microphone signals comprising the first sound and reflections of the first sound;
   generating at least one reflection parameter of the canal of the user's ear based on the first set of microphone signals;
   driving the speaker to project second sound into the canal of the user's ear;
   receiving a second set of microphone signals from the set of in-ear microphones, the second set of microphone signals comprising the second sound and reflections of the second sound; and

   generating a set of output signals based on the second set of microphone signals and the at least one reflection parameter; and
   transmitting a notification related to a characteristic of a hearing element of the user's ear based on the set of output signals.
18. The method of item 17, wherein generating the at least one reflection parameter comprises determining whether spectral content across a frequency band of a plurality of frequency bands of the first set of microphone signals primarily includes the first sound as direct sound from the speaker or the reflections of the first sound based on a comparison of the set of first microphone signals, wherein the at least one reflection parameter is generated in response to the spectral content including the reflections of the first sound.
19. The method of item 18, wherein determining whether the spectral content primarily includes the first sound as direct sound from the speaker or the reflections of the first sound comprises determining, over a period of time, whether the spectral content across the frequency band of the first set of microphone signals includes a threshold number of reflections of the first sound.
20. The method of any of items 17-19 further comprising
   determining whether the first sound exceeds a confidence threshold based on audio content of the first sound;
   in response to the first sound exceeds the confidence threshold, the notification related to the characteristic of the hearing element is transmitted.
21. The method of item 20, wherein determining whether the first sound exceeds the confidence threshold comprises determining whether the audio content includes at least one of a decay and a pause.
22. The method of any of items 17-21 further comprises
   determining a severity value of the characteristic based on the set of output signals; and
   determining a severity level of the characteristic based on a comparison between the severity value and one or more thresholds.
23. The method of any of items 17-22 further comprising estimating an ear geometry of the user's ear based on at least one of user input, sensor input, and the first set of microphone signals, wherein the characteristic of the hearing element is based on the estimated ear geometry of the user's ear.
24. The method of item 23 further comprising determining a severity level of the characteristic based on a comparison of the ear geometry and the generated set of output signals.

## Claims

1. A method performed by a headset that includes a speaker and a set of in-ear microphones, the method comprising:
driving, while the headset is being worn by a user, the speaker to project a first sound into a canal of a user's ear;
receiving a first set of microphone signals from the set of in-ear microphones, the first set of microphone signals comprising the first sound and reflections of the first sound;
generating at least one reflection parameter of the canal of the user's ear based on the first set of microphone signals;
driving the speaker to project second sound into the canal of the user's ear;
receiving a second set of microphone signals from the set of in-ear microphones, the second set of microphone signals comprising the second sound and reflections of the second sound;
generating a set of output signals based on the second set of microphone signals and the at least one reflection parameter; and
transmitting a notification related to a characteristic of a hearing element of the user's ear based on the set of output signals.

2. The method of claim 1, wherein generating the at least one reflection parameter comprises determining whether spectral content across a frequency band of a plurality of frequency bands of the first set of microphone signals primarily includes the first sound as direct sound from the speaker or the reflections of the first sound based on a comparison of the first set of microphone signals, wherein the at least one reflection parameter is generated in response to the spectral content including the reflections of the first sound.

3. The method of claim 2, wherein determining whether the spectral content primarily includes the first sound as direct sound from the speaker or the reflections of the first sound comprises determining, over a period of time, whether the spectral content across the frequency band of the first set of microphone signals includes a threshold number of reflections of the first sound.

4. The method of claim 3 further comprising using the first set of microphone signals to produce a directional beam pattern that is directed towards the canal of the user's ear and away from the headset, wherein the spectral content is of the directional beam pattern.

5. The method of claim 2, wherein each of the at least one reflection parameter is associated with a respective frequency band of the plurality of frequency bands, wherein generating the set of output signals comprises filtering, using the at least one reflection parameter, the second sound from the second set of microphone signals by removing spectral content across one or more frequency bands that are not associated with the at least one reflection parameter.

6. The method of claim 1 further comprising:
determining whether the first sound exceeds a confidence threshold based on audio content of the first sound; and
in response to the first sound exceeds the confidence threshold, the notification related to the characteristic of the hearing element is transmitted.

7. The method of claim 6, wherein determining whether the first sound exceeds the confidence threshold comprises determining whether the audio content includes at least one of a decay and a pause.

8. The method of claim 1 further comprises:
determining a severity value of the characteristic based on the set of output signals; and
determining a severity level of the characteristic based on a comparison between the severity value and one or more thresholds.

9. The method of claim 1 further comprising estimating an ear geometry of the user's ear based on at least one of user input, sensor input, and the first set of microphone signals, wherein the characteristic of the hearing element is based on the estimated ear geometry of the user's ear.

10. The method of claim 9 further comprising determining a severity level of the characteristic based on a comparison of the ear geometry and the generated set of output signals.

11. The method of claim 10, wherein the characteristic comprises a blockage within an ear canal of the user's ear and the ear geometry comprises a geometry of the ear canal, wherein the method further comprises estimating, using the set of output signals, a geometry of the blockage within the ear canal, wherein the severity level is based on differences between the geometry of the ear canal and the geometry of the blockage within the ear canal.

12. The method of claim 1, wherein the set of output signals comprise only reflections of the second sound captured by the second set of microphone signals.

13. The method of claim 1,
wherein driving the speaker to project the first sound comprises using a first portion of an input audio signal that comprises user-desired audio content to drive the speaker to project the first sound,
wherein driving the speaker to project the second sound comprises using a second, subsequent, portion of the input audio signal to drive the speaker to project the second sound.

14. The method of claim 1, wherein the set of in-ear microphones comprises at least two in-ear microphones.

15. A headset comprising:
at least one processor; and
memory coupled to the at least one processor, wherein the memory stores instructions that when executed by the at least one processor, cause the headset to perform the method of any one of claims 1-14.
